# EUROPEAN PATENT APPLICATION

(11) **EP 4 817 518 A1**
(43) Date of publication of application: **07.10.2026**
(21) Application number: 26168401.3
(22) Date of filing: 11.11.2021
(51) Int. Cl.: A61B 5/145, A61B 5/155

(54) **APPLICATOR FOR TRANSCUTANEOUS SENSOR AND APPLICATOR ASSEMBLY**

(30) Priority: 29.06.2021 KR 20210084618
(62) Document number(s) of the earlier application(s) in accordance with Art. 76 EPC: 21948572.9 / 4 344 629
(71) Applicant: I-SENS, INC., Seoul 06646 (KR)
(72) Inventor: CHOI, Hyun Ho, 06646 Seoul (KR); RYU, Goang Yel, 06646 Seoul (KR); WANG, Ji Hoon, 06646 Seoul (KR)
(74) Representative: BCKIP Part mbB

(57) **Abstract**

An applicator for inserting a sensor for biometric information measurement into a user's skin, according to the present invention, comprises: an applicator body which has a bottom portion capable of coming into contact with the user's skin; an insertion unit which is provided in the applicator body to move a sensor unit from a first position where the sensor unit is spaced apart from the user's skin to a second position where the sensor is inserted into the user's skin, the sensor unit comprising the sensor, a sensor unit housing in which the sensor is mounted, an adhesive layer provided on the sensor unit housing, and a protective sheet covering the adhesive layer; a carriage to which a part of the protective sheet is coupled and which is provided in the applicator body to move in the direction away from the sensor unit housing in order to release the protective sheet from the adhesive layer; and an operation member which is provided in the applicator body to be able to operate the carriage through the user's operation.

## Description

### TECHNICAL FIELD

The present disclosure relates to an applicator for a transcutaneous sensor, and more specifically, related to an applicator and an applicator assembly for inserting a transcutaneous sensor, which is inserted into the skin of a user to measure biometric information, into the skin of a user.

### BACKGROUND

With the recent advancement of medical technology, various medical devices that are attached to the body of a user have been developed and sold. Medical devices that are attached to the skin can be useful for monitoring biometric information or providing treatment by attaching them to the body of a patient with a chronic disease.

For example, chronic diseases such as diabetes require continuous management, and a body attachable unit for measuring biometric information can be used to manage blood glucose levels in diabetic patients. Diabetes is characterized by substantial absence of subjective symptoms at the beginning of the condition, when diabetes progresses, diabetes-specific symptoms such as overdrink, overeat, polyuria, weight loss, weariness, skin itchiness, and lower ability of naturally healing on injury on hands and feet are shown. Further progression of diabetes leads to complications such as visual disturbances, hypertension, kidney disease, paralysis, periodontal disease, muscle spasms and neuralgia, as well as gangrene. In order to diagnose diabetes beforehand and manage to prevent the progression of diabetes into complications associated therewith, systematic blood glucose measurement and treatment should be performed.

For diabetes patients as well as people having higher than normal blood glucose, even though diabetes has not yet developed, medical device manufacturers offer a variety of blood glucose meters to measure blood glucose levels.

Glucose measuring devices may be categorized into a single time measurement type measuring a blood glucose level and collecting blood from a fingertip by a user every single time and a continuous measurement type attaching a glucose monitoring system to the belly or an arm of the user and continuously measuring blood glucose levels.

Diabetics patients generally experience hyperglycemia and hypoglycemia, an emergency may occur in the hypoglycemic conditions, and the patients may become unconscious or die if a hypoglycemic condition lasts for an extended period of time without the supply of sugar. Accordingly, although rapid discovery of the hypoglycemic condition is critically important for diabetics, blood-collecting type glucose monitoring devices intermittently measuring glucose have limited ability to accurately measure blood glucose levels.

Recently, to overcome such a drawback, continuous glucose monitoring systems (CGMSs) inserted into the human body to measure a blood glucose level every few minutes have been developed, and therefore easily perform the management of diabetics and responses to an emergency situation.

A continuous blood glucose measurement system includes a body attachable unit that includes a transcutaneous sensor that is inserted into the skin of a user and measures blood glucose levels in the body, and a terminal that receives biometric information transmitted from the body attachable unit and outputs.

Systems for medical purposes using transcutaneous sensors are produced in many different forms by each manufacturer, and the methods of use also vary. Most systems currently manufactured and distributed are a method of attaching a disposable sensor unit to a body by an applicator. A user needs to perform several steps to attach the sensor unit to a skin using an applicator, and after attaching the sensor unit to a body, various follow-up procedures such as having to directly pull out the needle inserted into the skin along with the transcutaneous sensor are required to be performed.

For example, a user must peel off the packaging of the disposable sensor unit and accurately attach it to the applicator, and then insert the sensor unit into the applicator and operate the applicator to attach the sensor unit to a skin. Additionally, after attaching the sensor unit, there is the inconvenience of having to perform tasks such as pulling out the needle inserted into the skin and coupling the transmitter to the sensor unit.

### SUMMARY

### Technical Problem

The present disclosure is developed in consideration of the above-mentioned points, and the purpose of the present disclosure is to minimize the additional work to insert the transcutaneous sensor into a skin by enabling the sensor unit including the transcutaneous sensor to be manufactured in an assembled state, and to provide an applicator and applicator assembly for a transcutaneous sensor that can be conveniently used by a user to insert a transcutaneous sensor into the skin.

Another purpose of the present disclosure is to provide an applicator for a transcutaneous sensor and applicator assembly that can automatically separate the protective sheet that covers and protects the adhesive layer provided on the sensor unit.

### Solution to Problem

To accomplish the above-described purposes, an applicator for inserting a sensor for measuring biometric information into skin of a user according to an embodiment of the present disclosure may comprise: an applicator body having a bottom portion configured to be contactable with the skin of the user; an insertion unit installed to the applicator body to move the sensor unit, which comprises the sensor, a sensor unit housing to which the sensor is mounted, an adhesive layer comprised in the sensor unit housing, and a protective sheet covering the adhesive layer, from a first position where the sensor unit is spaced apart from the skin of the user to a second position where the sensor is inserted into the skin of the user; a carriage to which a part of the protective sheet is coupled and which is installed to the applicator body to move in a direction away from the sensor unit housing for releasing the protective sheet from the adhesive layer; and an operation member installed to the applicator body to operate the carriage by a manipulation of a user.

The carriage may be installed to move in a direction away from the bottom portion.

The carriage may be installed to move in a direction opposite to a movement direction of the sensor unit.

The applicator according to an embodiment of the present disclosure may comprise a carriage driver configured to move the carriage.

The applicator according to an embodiment of the present disclosure may comprise an elastic member configured to apply an elastic force to the carriage in a direction away from the bottom portion.

The applicator according to an embodiment of the present disclosure may furthur comprise a moving member installed to the applicator body to constrain movement of the carriage by engaging with the carriage such that the carriage maintains a third position in which the carriage is coupled to the protective sheet in a state in which the elastic member is elastically deformed, wherein the operating member is configured to operate to bias the moving member in a direction of being disengaged from the carriage.

The moving member may be installed to move in a direction intersecting the moving direction of the carriage.

The applicator according to an embodiment of the present disclosure may further comprise a return member elastically deformably installed to the applicator body and configured to apply an elastic force in a direction of being retreated with respect to the moving member by being elastically deformed by the moving member advanced by the operating member, wherein the carriage is configured to, as the moving member advances by the operating member, be disengaged from a first detent portion provided at one side of the moving member to move to a fourth position engaged with a second detent portion provided at an other side of the moving member, and, as the moving member retracts by the return member, be disengaged from the second detent to move to a fifth position separating the protective sheet from the adhesive layer.

The insertion unit may comprise a plunger installed movably from the first position to the second position together with the sensor unit, a needle separatably coupled to the sensor unit to be insertable into the skin of the user together with the sensor, a carrier installed to the plunger and coupled to the needle, and a plunger driver configured to provide a moving force in a direction of moving from the first position to the second position with respect to the plunger, and the plunger is configured to contact the moving member at the first position such that movement of the plunger is restricted.

A first moving distance by which the moving member advances to disengage the carriage from the first detent portion may be shorter than a second moving distance by which the moving member advances to disengage the plunger from the moving member.

The carriage may be configured to restrain a forward movement of the moving member to prevent the moving member from moving by the second moving distance in a state that the carriage is engaged with the second detent portion.

The plunger may be configured to be released from the moving member after a separation operation of the protective film by the carrier is completed.

The applicator body may include a column portion accommodating the plunger, the column portion has a column rail configured to guide the carriage to move linearly, and the elastic member is installed to connect the column portion and the carriage.

The applicator body may comprise a frame base portion having the bottom portion, a base frame protruding from the frame base portion and having a column portion accommodating the plunger, and a middle frame comprising a stage arranged at an upper side of the frame base and supporting the moving member, and a middle frame opening formed at a middle of the stage to allow the column portion to be inserted.

The applicator according to an embodiment of the present disclosure may further comprise a needle release driver configured to provide a moving force to the carrier such that the carrier moves the needle in a direction away from the skin of the user after the needle is inserted into the skin of the user with the sensor.

The protective sheet may comprise a protection portion covering the adhesive layer, and a wing portion extending from an edge of the protection portion to be coupled to the carriage.

The protective sheet may have a protective sheet groove, and the carriage may comprise a grip portion inserted into the protective sheet groove.

The carriage may be configured to operates to complete a separation operation of the protective sheet before the sensor unit moves from the first position.

Additionally, to accomplish the above-described purposes, an applicator for inserting a sensor for measuring biometric information into skin of a user according to another embodiment of the present disclosure may comprise: an applicator body having a bottom portion configured to be contactable with the skin of the user; an insertion unit installed to the applicator body to move the sensor unit, which comprises the sensor, a sensor unit housing to which the sensor is mounted, an adhesive layer comprised in the sensor unit housing, and a protective sheet covering the adhesive layer, from a first position where the sensor unit is spaced apart from the skin of the user to a second position where the sensor is inserted into the skin of the user; and a grip portion installed to the applicator body such that a portion of the protective sheet is coupled, wherein the protective sheet is separated from the sensor unit housing by changing a distance between the sensor unit housing and the grip portion.

Additionally, to accomplish the above-described purposes, an applicator for inserting a sensor into skin of a user according to still another embodiment of the present disclosure may comprise: an applicator body to which a base unit comprising a base unit housing and an adhesive portion provided on the base unit housing to be attached to the skin of the user is separatably coupled, the applicator body having a bottom portion configured to be contactable with the skin of the user; an insertion unit installed to the applicator body to move the sensor unit, which comprises the sensor, a sensor unit housing to which the sensor is mounted, an adhesive layer comprised in the sensor unit housing to be adhered to the base unit housing, and a protective sheet covering the adhesive layer, from a first position where the sensor unit is spaced apart from the base unit to a second position where the sensor unit is coupled to the base unit such that the sensor is inserted into the skin of the user; a carriage to which a part of the protective sheet is coupled and which is installed to the applicator body to move in a direction away from the bottom portion for releasing the protective sheet from the adhesive layer; and an operation member installed to the applicator body to operate the carriage by a manipulation of a user.

The grip portion may be installed to the applicator body such that the grip portion is movable in a direction away from the sensor unit housing.

The grip portion may be fixed at a position spaced apart from the bottom portion.

Additionally, an applicator assembly according to an embodiment of the present disclosure may comprise: a sensor unit comprising a sensor, a sensor unit housing to which the sensor is mounted, an adhesive layer comprised in the sensor unit housing, and a protective sheet covering the adhesive layer; an applicator body having a bottom portion configured to be contactable with skin of an user; an insertion unit installed to the applicator body to move the sensor unit from a first position where the sensor unit is spaced apart from the skin of the user to a second position where the sensor unit is inserted into the skin of the user; a carriage to which a part of the protective sheet is coupled and which is installed to the applicator body to move in a direction away from the bottom portion for releasing the protective sheet from the adhesive layer; and an operation member installed to the applicator body to operate the carriage by a manipulation of a user.

The applicator assembly according to an embodiment of the present disclosure may further comprise a base unit comprising a base unit housing and an adhesive portion provided on the base unit housing to be attached to the skin of the user, wherein the base unit separatably coupled to the applicator body, wherein the sensor unit housing is attached to the base unit housing by the adhesive layer at the second position.

### Advantageous Effects of Invention

According to the present disclosure, by manufacturing the sensor unit in an assembled state within the applicator, the additional work for a user to attach the sensor unit to the skin of a user is minimized, and the sensor unit can be attached to the skin of a user simply by operating the applicator.

Additionally, according to the present disclosure, by covering and protecting the adhesive layer of the sensor unit with a protective sheet, it may prevent the problem of deterioration of the adhesiveness of the adhesive layer before inserting the sensor into the skin of a user.

Additionally, according to the present disclosure, the protective sheet that covers and protects the adhesive layer of the sensor unit can be automatically separated by operating the applicator. Therefore, no additional work is required to separate the adhesive layer of the sensor unit, and convenience of use is excellent.

### BRIEF DESCRIPTION OF THE DRAWINGS

FIG. 1 is a perspective view showing an applicator assembly according to an embodiment of the present disclosure.
FIG. 2 shows a body attachable unit attached to the skin of a user.
FIG. 3 is a perspective view showing a body attachable unit.
FIGS. 4 and 5 are exploded perspective views showing a sensor unit.
FIG. 6 is a cross-sectional view showing a portion of a sensor unit disassembled.
FIG. 7 is a cross-sectional view showing a needle coupled to a sensor unit.
FIG. 8 is a cross-sectional view showing a needle being separated from a sensor unit.
FIG. 9 shows a protective sheet attached to an adhesive layer of a sensor unit.
FIG. 10 shows the appearance after the protective sheet is separated from an adhesive layer of a sensor unit.
FIGS. 11 and 12 show a sensor unit and a base unit before they are coupled.
FIG. 13 is a cross-sectional view showing a body attachable unit assembled by coupling a sensor unit and a base unit.
FIG. 14 is a cross-sectional view taken along line I-I in FIG. 1.
FIG. 15 is a cross-sectional view taken along line II-II in FIG. 1.
FIG. 16 shows the top case of an applicator according to an embodiment of the present disclosure separated from an applicator body.
FIG. 17 shows a protective cap and safety pin of an applicator according to an embodiment of the present disclosure separated from an applicator body.
FIGS. 18 and 19 are exploded perspective views of an applicator according to an embodiment of the present disclosure.
FIGS. 20 and 21 are perspective views showing a needle assembly of an applicator according to an embodiment of the present disclosure.
FIGS. 22 and 23 are perspective views showing a needle.
FIG. 24 is a cross-sectional view showing a carrier and a needle before being coupled.
FIG. 25 is a cross-sectional view showing a carrier and a needle coupled.
FIG. 26 is a perspective view showing a state in which a locking hook is engaged with a base unit housing by a moving member.
FIG. 27 is a front view showing a state in which a locking hook is engaged with a base unit housing by a moving member.
FIG. 28 is a perspective view showing a state in which a locking hook is disengaged from a base unit housing by a moving member.
FIG. 29 is a front view showing the unlocked state in which a locking hook is engaged with a base unit housing by a moving member.
FIGS. 30 and 31 are exploded views of some components of an applicator according to an embodiment of the present disclosure.
FIGS. 32 to 44 show a process in which a sensor of a sensor unit is inserted into the skin of a user by an applicator according to an embodiment of the present disclosure.
FIG. 45 is a cross-sectional view showing a portion of an applicator assembly according to another embodiment of the present disclosure.
FIG. 46 shows a sensor unit of an applicator assembly shown in FIG. 45.
FIGS. 47 and 48 show another embodiment of a sensor unit and a base unit.

### DETAILED DESCRIPTION OF EMBODIMENTS

Hereinafter, the applicator and applicator assembly for a transcutaneous sensor according to the present disclosure will be described in detail with reference to the drawings.

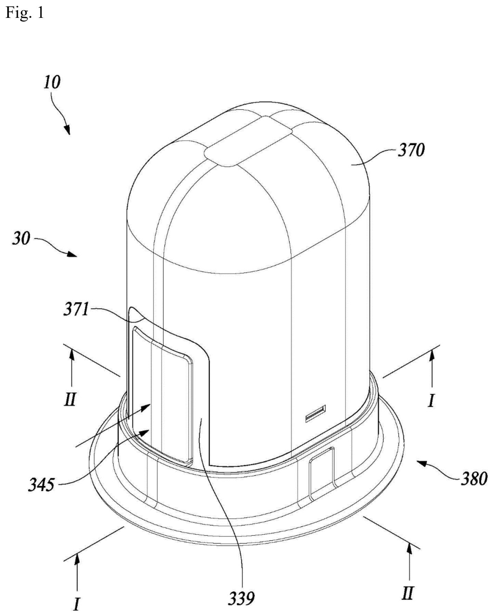

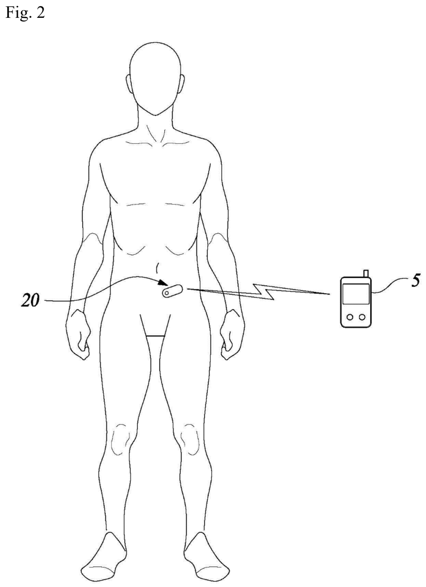

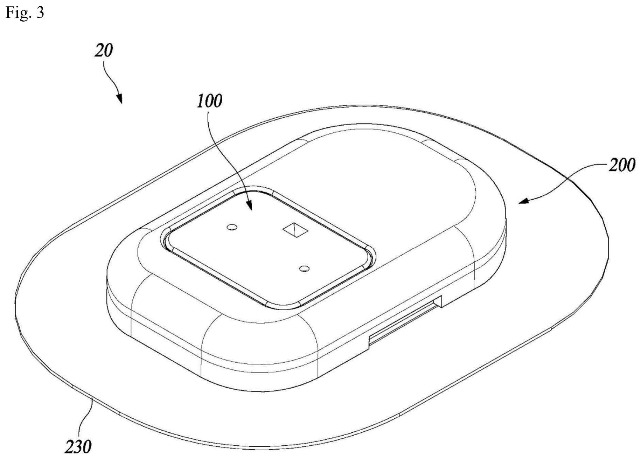

FIG. 1 is a perspective view showing an applicator assembly according to an embodiment of the present disclosure, FIG. 2 is showing a body attachable unit attached to the skin of a user, and FIG. 3 is a perspective view showing a body attachable unit.

An applicator assembly (10) according to an embodiment of the present disclosure includes a sensor unit (100) including a sensor (110) that is inserted into the skin of a user and measures biometric information, and a base unit (200) that is coupled with the sensor unit (100) to form a body attachable unit (20), and an applicator (30) for attaching the body attachable unit (20) to the skin of a user. The applicator assembly (10) may be provided to a user with the sensor unit (100) and the base unit (200) mounted separately on the applicator (30). The sensor unit (100) and the base unit (200) can be automatically assembled and attached to the skin of a user by the operation of the applicator (30).

As shown in FIG. 2, the body attachable unit (20) can be attached to the skin of a user by the applicator (30), measure biometric information, and wirelessly transmit the measured data to an external terminal (5). The external terminal (5) may be a variety of devices capable of receiving measurement data from the body attachable unit (20), such as a portable terminal, a medical device, a PC, or a server. Biometric information that the body attachable unit (20) can measure is not limited to specific information. As an exemplary embodiment, the body attachable unit (20) may periodically measure blood glucose level for a user and transmit the blood glucose measurement information to the external terminal (5).

As shown in FIG. 3, the body attachable unit (20) includes the sensor unit (100) for measuring the biometric information of a user, and the base unit (200) that is attached to the skin of a user and coupled to the sensor unit (100). do. The base unit (200) may have electronic components installed therein. This base unit (200) is electrically connected to the sensor unit (100) and can process biometric information measured by the sensor unit (100) and transmit it to the external terminal (5).

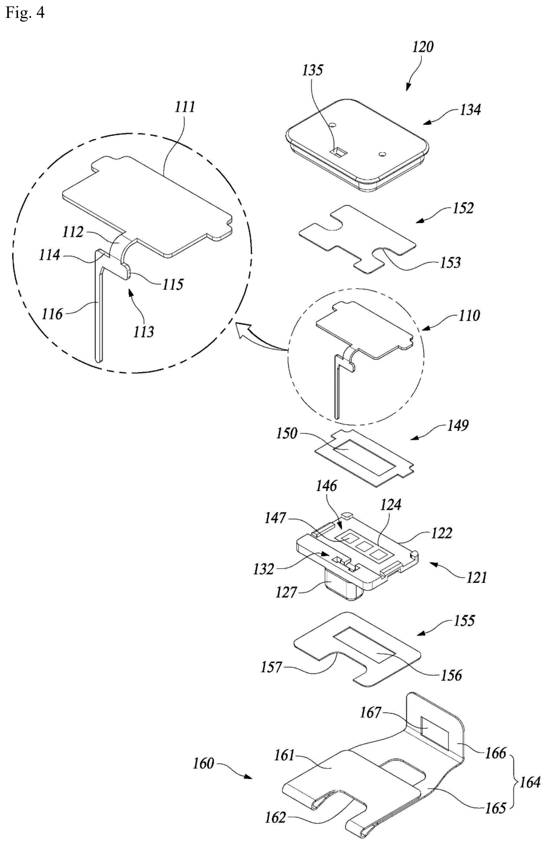

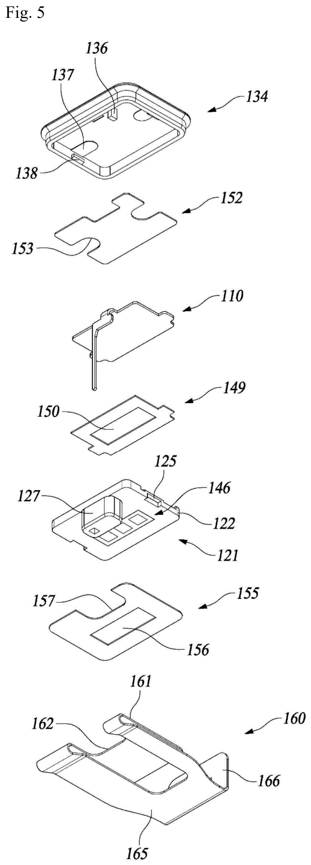

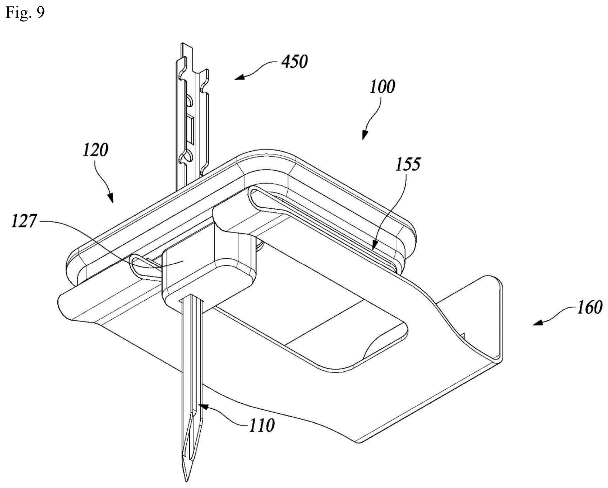

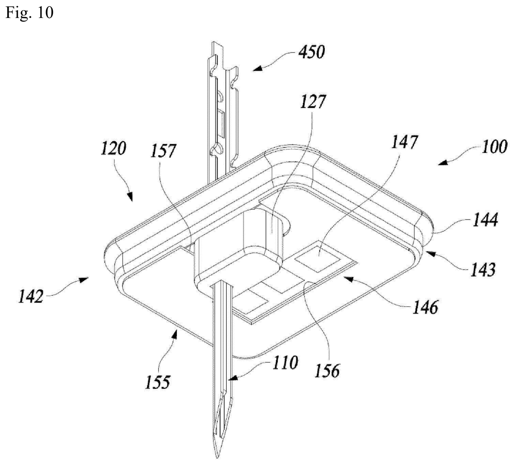

As shown in FIGS. 4 to 10, the sensor unit (100) includes the sensor (110) inserted into the skin of a user, a sensor unit housing (120) to which the sensor (110) is coupled, a sensor unit-electrical contact portion(146) electrically connected to the sensor (110) coupling to the sensor unit housing(120), a sensor adhesive portion (149) and an adhesive pad (152) for fixing the sensor (110) to the sensor unit housing (120), an adhesive layer (155) provided on the sensor unit housing (120) so as to be attached to the base unit (200), a protective sheet (160) covering the adhesive layer (155).

The sensor (110) includes a sensor body (111), a connection portion (112) connected to one side of the sensor body (111), a middle portion (113) connected to the sensor body (111) through the connection portion (112), and an insertion portion (116) connected to the middle portion (113) so as to be inserted into the skin of a user. The sensor body (111) is disposed inside the sensor unit housing (120) so as to be in contact with the sensor unit-electrical contact portion (146). The sensor body (111) may be provided with an electrode electrically connected to the sensor unit-electrical contact portion (146). The connection portion (112) extends from the edge of the sensor body (111) and connects the sensor body (111) and the middle portion (113) to lie on different planes. The sensor (110) may be manufactured in a flat form in which the sensor body (111) lies on the same plane as the middle portion (113) and the insertion portion (116), and then manufactured in a form in which the connection portion (112) is bent and deformed so that the sensor body (111) lies on a different plane from the middle portion (113) and the insertion portion (116).

The middle portion (113) and the insertion portion (116) are connected to the sensor body (111) so as to lie on a different plane from the sensor body (111). In the drawing, the middle portion (113) and the insertion portion (116) are shown to be disposed on the same plane, and the middle portion (113) is disposed perpendicular to the sensor body (111), but the angle between the middle portion (113) and the sensor body (111) or the angle between the insertion portion (116) and the sensor body (111) can be changed in various ways.

The middle portion (113) includes a first extension portion (114) that protrudes to one side of the connection portion (112), and a second extension portion (115) that protrudes from the connection portion (112) in a direction opposite to the direction in which the first extension portion (114) protrudes from the connection portion (112). The insertion portion (116) may be connected to the first extension portion (114) and inserted into the skin of a user. The insertion portion (116) has a relatively thin and long shape so as to be smoothly inserted into the skin of a user. The connection portion (112), the middle portion (113), and the insertion portion (116) may be provided with a conductive trace electrically connected to the electrode of the sensor body (111). The sensor (110) may be coupled to the sensor unit housing (120) so that the sensor body (111), the connection portion (112) and the middle portion (113) are located inside the sensor unit housing (120) and only the insertion portion (116) protrudes from the sensor unit housing (120).

The sensor unit housing (120) includes a housing base (121) and a housing cap (134) that covers the top of the housing base (121). A space is provided inside the sensor unit housing (120) to accommodate the sensor (110), and a housing opening (140) into which a needle (450) for inserting the sensor (110) into the skin of a user is inserted is formed in the middle of the sensor unit housing (120) to penetrate the sensor unit housing (120) in the thickness direction.

The housing base (121) includes a base portion (122) supporting the sensor body (111) of the sensor (110), and a boss (127) protruding from the surface of the base portion (122). On one side of the base portion (122), a base portion hole (123) is formed to penetrate the base portion (122), and on the other side of the base portion (122), a through hole (124) is formed to penetrate the base portion (122). A sensor unit-electrical contact portion (146) is coupled to the through hole (124). A detent groove (125) is provided at the edge of the base portion (122).

The boss (127) protrudes from the surface on the side of the base portion (122) on which the sensor (110) is not placed. A boss hole (128) is formed in the boss (127) to penetrate the boss (127). The boss hole (128) is connected to the base portion hole (123) and forms a housing base hole (132) together with the base hole (123). The boss hole (128) includes an upper hole (129) connected to the base portion hole (123) and a lower hole (130) connected to the upper hole (129) and extending to the end of the boss (127). The size of the upper hole (129) is larger than the size of the lower hole (130). The upper hole (129) is sized to accommodate the middle portion (113) of the sensor (110) and portion of the needle (450), and the lower hole (130) is sized to accommodate the insertion portion (116) of the sensor (110) and a portion of the needle (450). The upper hole (129) is provided with a support portion (131) capable of supporting the middle portion (113) of the sensor (110). As the support portion (131) supports the middle portion (113), the sensor (110) does not move while being coupled to the sensor unit housing (120) and can maintain a stable coupling state with the sensor unit housing (120).

The housing cap (134) is coupled to the housing base (121) and covers the sensor body (111) of the sensor (110) placed on the housing base (121) and the upper part of the housing base (121). A housing cap hole (135) is formed in the middle of the housing cap (134) to penetrate the housing cap (134) in the thickness direction. The housing cap hole (135) is connected to the housing base hole (132) of the housing base (121) to form the housing opening (140). The needle (450) for inserting the sensor (110) into the skin of a user is inserted along with the sensor (110) into the housing opening (140). The housing cap (134) is provided with a detent protrusion (138) corresponding to the detent groove (125) of the housing base (121). The housing cap (134) may be coupled to the housing base (121) by inserting the detent protrusion (138) into the detent groove (125) and engaging the housing base (121). A protrusion (137) and a retention protrusion (136) are provided on the inner surface of the housing cap (134) facing the housing base (121). The protrusion (137) and the retention protrusion (136) protrude toward the housing base (121). The protrusion (137) and the retention protrusion (136) may contact the sensor (110) to prevent movement of the sensor (110) and stably fix the sensor (110) to the sensor unit housing (120). The protrusion (137) may contact the sensor body (111) of the sensor (110) and press the sensor body (111) toward the housing base (121). Therefore, the sensor body (111) can remain stably fixed to the housing base (121) without being lifted from the housing base (121).

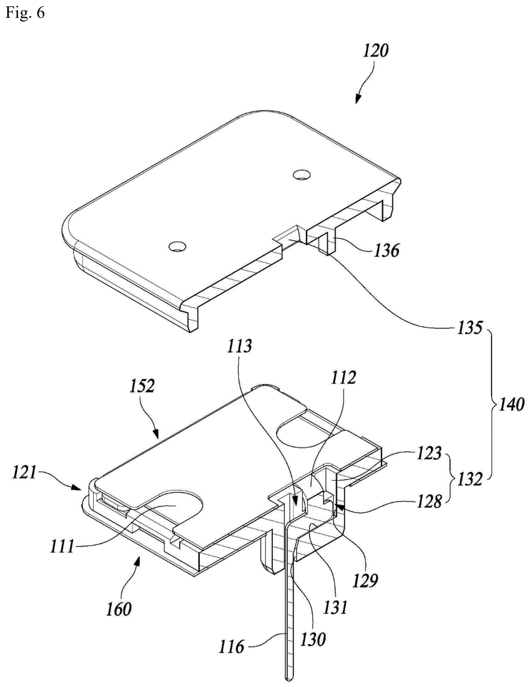

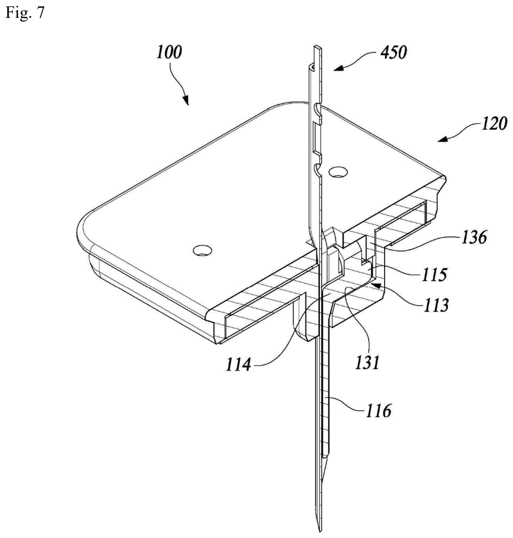

As shown in FIGS. 6 and 7, when the housing cap (134) is coupled to the housing base (121), the retention protrusion (136) is inserted into the housing base hole (132) and comes into contact with the sensor (110). That is, the retention protrusion (136) is arranged so that its end faces the second extension (115) of the middle portion (113) inserted into the housing base hole (132), and may restrain the movement of the middle portion (113) of the sensor (110) so that the middle portion (113) does not lift off the support portion (131). Due to the action of these retention protrusions (136), the insertion portion (116) of the sensor (110) can be stably maintained in a state of protruding from the sensor unit housing (120) by a certain length. And, due to the action of the retention protrusion (136), the insertion portion (116) of the sensor (110) does not retreat from the skin of a user while being inserted into the skin of a user, and can remain inserted at a certain depth into the skin of a user.

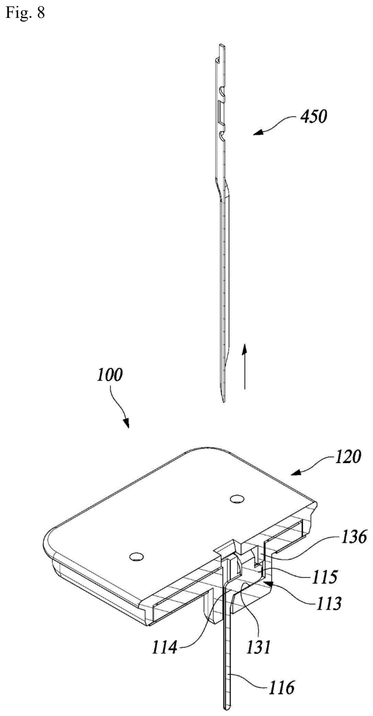

Also, as shown in FIG. 8, after the insertion portion (116) is inserted into the skin of a user together with the needle (450), the retention protrusion (136) prevents the middle portion (113) from moving during the process of the needle (450) coming out of the skin of a user, so that the insertion portion (116) cannot retract in the same direction as the needle (450). Accordingly, it is possible to prevent a problem in which the insertion portion (116) inserted into the skin of a user moves in the direction in which the needle (450) exits the skin of a user during the process of the needle (450) coming out of the skin of a user. In addition, it may prevent the problem of the insertion portion (116) partially coming out of the skin and reducing the measurement accuracy of the sensor (110).

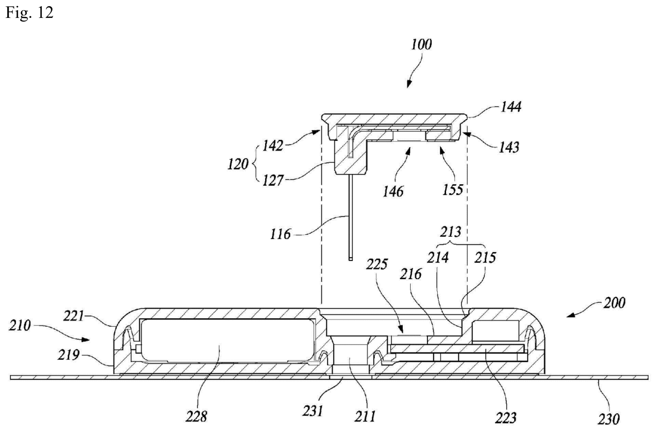

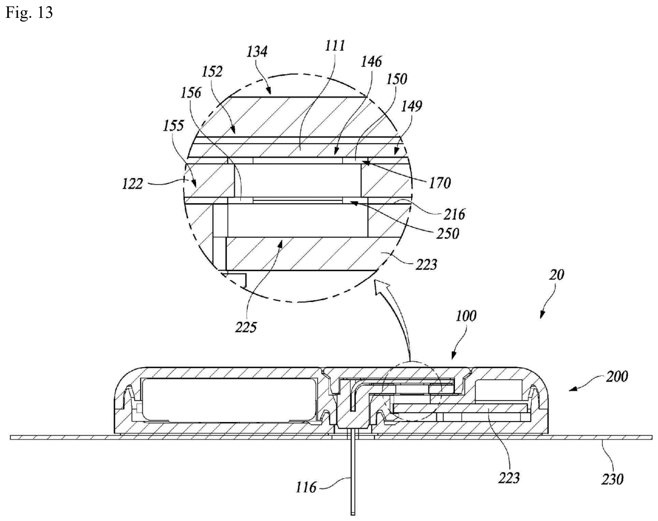

The housing cap (134) forms the housing body (142) together with the base portion (122). That is, the sensor unit housing (120) may be configured to include a housing body (142) and a boss (127) protruding from one side of the housing body (142). As shown in FIGS. 12 and 13, the housing body (142) is shaped to fit into a base unit recess (213) of the base unit (200). The housing body (142) includes a body portion (143) provided with a boss (127) and a cover portion (144) that is wider than the body portion (143).

The sensor unit housing (120) is not limited to the configuration shown, and may be changed to various other configurations on which the sensor (110) may be mounted and coupled to the base unit (200). That is, the sensor unit housing (120) includes a housing base (121) and the housing cap (134), but it can be changed to various configurations other than a configuration including a housing body (142) and a boss (127) protruding from the housing body (142).

The sensor unit-electrical contact portion (146) is disposed in the sensor unit housing (120) to be electrically connected to the sensor body (111) of the sensor (110). The sensor unit-electrical contact portion (146) is inserted into the through hole (124) of the base portion (122) and is disposed to vertically penetrate the base portion (122). The sensor unit-electrical contact portion (146) may include a plurality of terminal portions (147) for transmitting electrical signals. Some part of the sensor unit-electrical contact portion (146) is exposed to one surface of the base portion (122) where the sensor body (111) of the sensor (110) is located, and another part is exposed to the other side of the base portion (122), so that the sensor (110) and a base unit- electrical contact portion (225) of the base unit (200) can be electrically connected.

The sensor adhesive portion (149) is disposed between the housing base (121) and the sensor body (111) of the sensor (110) to attach the sensor body (111) to the housing base (121). The sensor adhesive portion (149) has adhesive properties on both sides. That is, one side of the sensor adhesive portion (149) is adhered to the housing base (121), and the other surface of the sensor adhesive portion (149) is adhered to the sensor body (111). A sensor adhesive portion opening (150) is formed in the middle of the sensor adhesive portion (149) to penetrate the sensor adhesive portion (149) in the thickness direction. The sensor body (111) of the sensor (110) is in contact with the sensor unit-electrical contact portion (146) through the sensor adhesive portion opening (150). Therefore, the sensor adhesive portion (149) seals between the sensor body (111) and the sensor unit-electrical contact portion (146), thereby preventing moisture or foreign substances from entering an electrical connection portion (170) between the sensor body (111) and the sensor unit electrical contact portion (146).

The shape of the sensor adhesive portion (149) is not limited to that shown and can be changed in various ways.

The adhesive pad (152) is disposed between the housing cap (134) and the sensor body (111) of the sensor (110) to attach the sensor body (111) to the housing cap (134). The adhesive pad (152) has adhesive properties on both sides. That is, one side of the adhesive pad (152) is adhered to the housing cap (134), and the other side of the adhesive pad (152) is adhered to the sensor body (111). An adhesive pad groove (153) is formed in the adhesive pad (152) to penetrate the adhesive pad (152) in the thickness direction. The protrusion (137) of the housing cap (134) contacts the sensor body (111) through the adhesive pad groove (153), thereby pressing the sensor body (111) toward the housing base (121).

The shape of the adhesive pad (152) is not limited to that shown and can be changed in various ways.

An adhesive layer (155) is disposed on the surface of the housing base (121). The adhesive layer (155) has adhesive properties on both sides. One side of the adhesive layer (155) is adhered to the housing base (121), and the other side of the adhesive layer (155) is covered with the protective sheet (160). The adhesive layer (155) may be attached to the base unit (200) after the protective sheet (160) is separated. An adhesive layer hole (156) is formed in the middle portion of the adhesive layer (155) to penetrate the adhesive layer (155) in the thickness direction. The sensor unit-electrical contact portion (146) may contact the base unit-electrical contact portion (225) of the base unit (200) through the adhesive layer hole (156). Therefore, the adhesive layer (155) seals between the sensor unit-electrical contact portion (146) and the base unit-electrical contact portion (225), thereby preventing moisture or foreign substances from entering the electrical connection portion (250) between the sensor unit (100) and the base unit (200). An adhesive layer opening (157) is formed on one side of the adhesive layer (155) to penetrate the adhesive layer (155) in the thickness direction. The adhesive layer (155) is attached to the housing base (121) such that the sensor unit-electrical contact portion (146) is located inside the adhesive layer hole (156) and the boss (127) is inserted into the adhesive layer opening (157).

The shape of the adhesive layer (155) is not limited to that shown and can be changed in various ways.

The protective sheet (160) covers and protects the adhesive layer (155). The protective sheet (160) is made of a material that is detachably attached to the adhesive layer (155). If the adhesive layer (155) is left exposed to the air for a long time, the adhesiveness of the adhesive layer (155) may deteriorate. The protective sheet (160) covers the adhesive layer (155) to prevent the problem of reducing of adhesiveness of the adhesive layer (155), and it facilitates handling of the sensor unit (100) by an operator during the manufacturing process of the sensor unit (100) or the process of assembling the sensor unit (100) to the applicator (30). The protective sheet (160) includes a protection portion (161) adhered to the adhesive layer (155) and a wing portion (164) extending from an edge of the protection portion (161). A protective sheet opening (162) is formed on one side of the protection portion (161) to penetrate the protection portion (161) in the thickness direction. The protective sheet (160) is attached to the adhesive layer (155) such that the boss (127) is inserted into the protective sheet opening (162). The length of the wing portion (164) is longer than the length of the protection portion (161). The wing portion (164) may be bent at one end of the protection portion (161) to completely cross the protection portion (161) and extend outward from the other end of the protection portion (161). The wing portion (164) includes a first wing portion (165) that overlaps the protection portion (161) and a second wing portion (166) that is bent at the first wing portion (165). The protective sheet opening (162) may extend from the protection portion (161) to the first wing portion (165). A protective sheet groove (167) is formed in the second wing portion (166) to penetrate the second wing portion (166) in the thickness direction. The wing portion (164) may extend a certain distance from the edge of the sensor unit housing (120) and be coupled to the carriage (610) of the applicator (30). The protective sheet (160) may be separated from the adhesive layer (155) before the sensor unit (100) moves and reaches the base unit (200).

The shape of the protective sheet (160) is not limited to that shown and can be changed in various ways.

The sensor unit (100) is mounted on the applicator (30) with the protective sheet (160) attached to the adhesive layer (155). The protective sheet (160) may be separated from the adhesive layer (155) by a removing unit (600) of the applicator (30) before the sensor (110) is inserted into the skin of a user. The sensor unit (100) moves toward the base unit (200) with the protective sheet (160) separated and is coupled to the base unit (200).

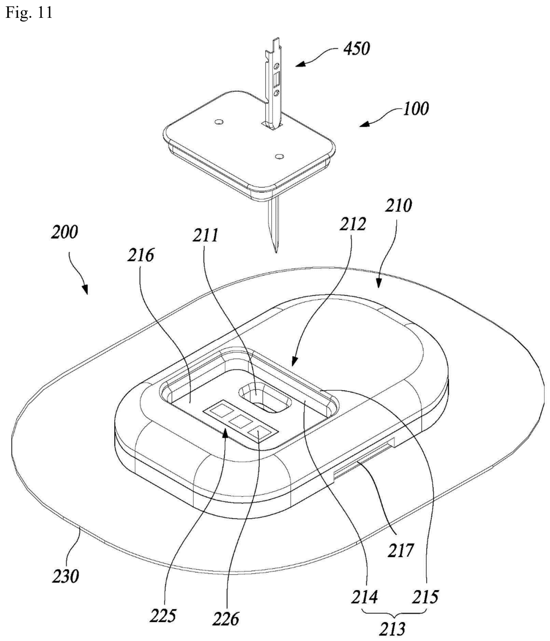

As shown in FIGS. 11 to 13, the base unit (200) includes a base unit housing (210) to which the sensor unit (100) is coupled, and electronic components installed inside the base unit housing (210). The electronic component may include a circuit board (223), a base unit electrical contact portion (225) in contact with the sensor unit-electrical contact portion (146) of the sensor unit (100), a battery (228), etc. The circuit board (223) may be equipped with a processor chip for processing signals, a communication chip for wireless communication with the external terminal 5, etc.

The base unit housing (210) is provided with an insertion hole (211) through which the insertion portion (116) of the sensor (110) and the needle (450) can pass, and a mounting portion (212) in which the sensor unit housing (120) is coupled. The mounting portion (212) includes a base unit recess (213) and a contact surface (216) provided inside the base unit recess (213). The insertion hole (211) is shaped so that the boss (127) of the sensor unit (100) can be fitted and is disposed inside the base unit recess (213). The contact surface (216) may be flat so that the adhesive layer (155) of the sensor unit (100) can be stably adhered. The base unit recess (213) includes a first recess (214) connected to the insertion hole (211), and a second recess (215) that is located farther from the insertion hole (211) than the first recess (214) and is connected to the first recess (214). The second recess (215) is wider than the first recess (214). The first recess (214) can be made in a shape corresponding to the body portion (143) of the sensor unit housing (120), and the second recess (215) can be made in a shape corresponding to the cover portion (144) of the sensor unit housing (120). Therefore, the sensor unit housing (120) can be fitted into the base unit recess (213) and maintain a stable coupling state with the base unit housing (210). In addition, since the sensor unit housing (120) is stably fitted and coupled to the base unit housing (210), moisture or foreign substances cannot easily enter the gap between the sensor unit housing (120) and the base unit housing (210). A housing groove (217) is formed on the outer edge of the base unit housing (210). Some part of a locking hook (360) provided on the applicator (30) may be inserted into the housing groove (217).

The base unit-electrical contact portion (225) is arranged on the mounting portion (212) and contacts the sensor unit-electrical contact portion (146) of sensor unit (100) when sensor unit (100) is coupled to the base unit (200). The base unit-electrical contact portion (225) is electrically connected to the circuit board (223) and is installed in the base unit housing (210) so that a portion is exposed to the base unit recess (213). The base unit-electrical contact portion (225) may include a plurality of terminal portions (226) for transmitting electrical signals. The terminal portion (226) may electrically connect the sensor unit-electrical contact portion (146) and the circuit board (223) by contacting the terminal portion (147) of the sensor unit (100).

As shown, the base unit housing (210) may include a lower housing (219) and an upper housing (221) that covers the upper part of the lower housing (219), but its shape may be changed in various ways.

An adhesive portion (230) is provided on the surface of the base unit housing (210). The adhesive portion (230) is attached to the surface of the lower housing (219) to adhere the base unit housing (210) to the skin of a user. In the middle of the adhesive portion (230), an adhesive hole (231) through which the insertion portion (116) of the sensor (110) and the needle (450) can pass is formed to penetrate the adhesive portion (230) in the thickness direction.

The adhesive portion (230) may be covered and protected with a protective sheet. The protective sheet covering the adhesive portion (230) may be removed during the process of attaching the base unit (200) to the skin of a user.

The sensor unit (100) and the base unit (200) are installed in the applicator (30) in a separated state, and are coupled to each other in the process of operating the applicator (30) to insert the sensor (110) into the skin of a user, forming the body attachable unit (20). The sensor unit (100) may be mounted on the applicator (30) while the needle (450) coupled, and moved toward the base unit (200) with the needle (450) to be coupled to the base unit (200). After the sensor unit (100) is coupled to the base unit (200), the needle (450) is separated from the sensor unit (100), and only the body attachable unit (20) remains on the skin of a user.

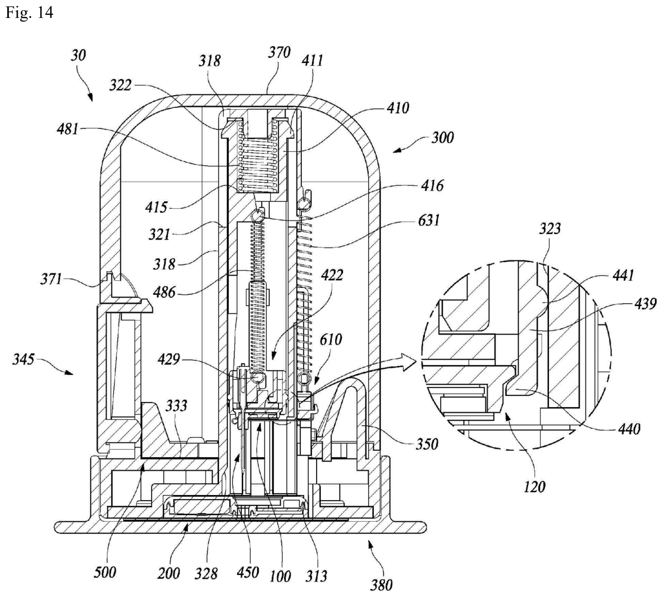

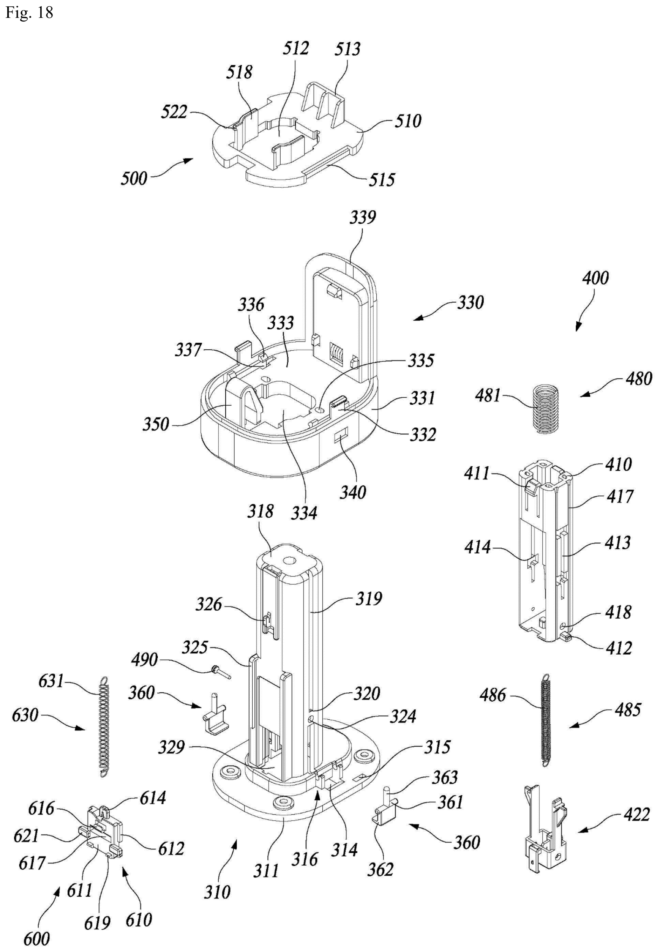

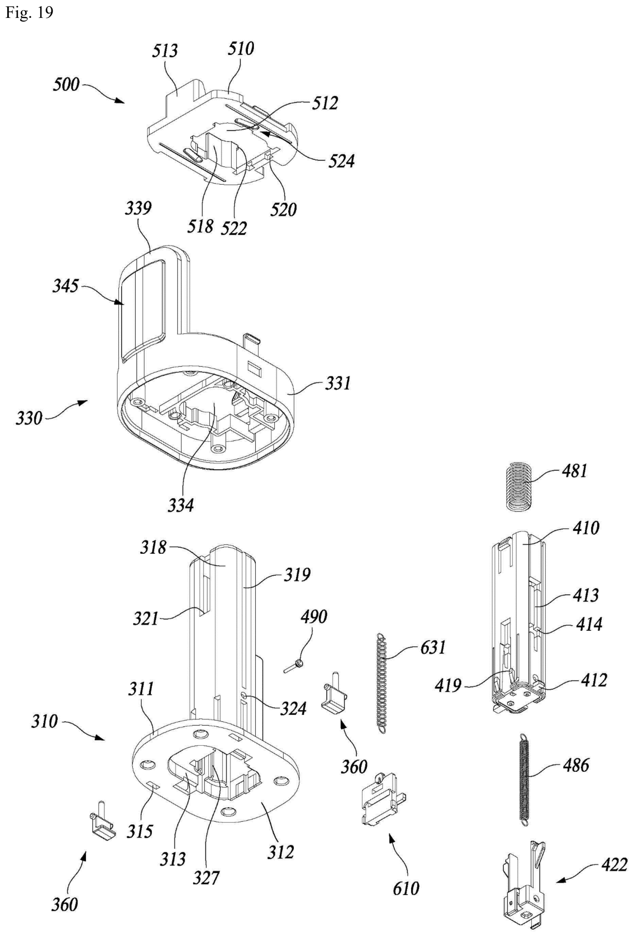

Referring to FIGS. 14 to 19, the applicator (30) operates with the sensor unit (100) and the base unit (200) mounted, thereby coupling the sensor unit (100) and the base unit (200) and can attach the body attachable unit (20) in which the sensor unit (100) and the base unit (200) are coupled to the skin of a user. The sensor unit (100) and the base unit (200) are spaced apart from each other and are respectively mounted on the applicator (30). The applicator (30) is separated from the body attachable unit (20) after attaching the body attachable unit (20) to the skin of a user, and the body attachable unit (20) remains attached to the skin of a user by the adhesive portion (230).

The applicator (30) includes an applicator body (300) to which the base unit (200) is detachably coupled, a protective cap (380) which is detachably coupled to the applicator body (300), an insertion unit (400) that moves the sensor unit (100) to insert the sensor (110) into the skin of a user, a moving member (500) installed movably on the applicator body (300), and a removing unit (600) for separating the protective sheet (160) of the sensor unit (100). The insertion unit (400) may move the sensor unit (100) from a first position spaced apart from the base unit (200) by a preset distance to a second position coupled with the base unit (200). The sensor unit (100) and the base unit (200) are coupled at the second position to form the body attachable unit (20).

The applicator body (300) includes a base frame (310) on which the insertion unit (400) is installed, a middle frame (330) disposed on the base frame (310), and a top case (370) that covers the upper part, coupling to the middle frame (330).

The base frame (310) includes a frame base portion (311) having a bottom portion (312) that can be in contact with the skin of a user, a column portion (318) that protrudes from the frame base portion (311) and accommodates the sensor unit (100) and a plunger (410) of the insertion unit (400).

The bottom portion (312) of the frame base portion (311) is provided with a recess (313) into which the base unit (200) is mounted. The base unit (200) is detachably coupled to the recess (313) so that the adhesive portion (230) can face the skin of a user and placed in a second position spaced apart from the sensor unit (100). A plurality of through holes (314 and 315) are formed in the frame base portion (311) to penetrate the frame base portion (311) in the thickness direction. Among these, one pair of through holes (314) are disposed on both sides of the column portion (318) with the column portion (318) in between, and the other through hole (315) is disposed on the other side of the frame base portion (311). A support portion (316) is provided at the top of each of a pair of through holes (314) disposed on both sides of the column portion (318), and the stopper arm (384) of the protective cap (380) can be inserted into the other through hole (315). The stopper arm (384) is used to restrict the movement of the moving member (500), and its specific operation will be described later.

A locking hook (360) for detachably fixing the base unit (200) is coupled to the support portion (316). The locking hook (360) may engage the base unit (200) mounted in the recess (313) and secure the base unit (200) without being separated from the recess (313). The locking hook (360) may be disengaged from the base unit (200) after the body attachable unit (20) is attached to the skin of a user. The locking hook (360) is pivotally coupled to the support portion (316). The locking hook (360) includes a pivot portion (361) rotatably coupled to the support portion (316), a hook portion (362) connected to the pivot portion (361) and a rod (363) protruding from the pivot portion (361). The hook portion (362) protrudes from one side of the pivot portion (361), and the rod (363) protrudes from the other side of the pivot portion (361). The hook portion (362) is inserted into the through hole (314) of the base frame (310), and a portion of it is located in the recess (313) of the base frame (310). The locking hook (360) may be disengaged from the base unit (200) in conjunction with the moving member (500). A more specific operation of the locking hook (360) will be described later.

Slits (319) are formed on both sides of the column portion (318) in a direction parallel to the moving direction of the sensor unit (100). A release portion (320) is provided in the middle portion of the slit (319). The release portion (320) is a part of a carrier (422) disposed inside the plunger (410) that can be contacted while the carrier (422) moves toward the base unit (200). The carrier (422) may be disengaged from the plunger (410) by the action of the release portion (320). The specific operation of the release portion (320) will be described later. A detent portion (321) is provided on one side of the column portion (318) to limit the moving distance of the plunger (410). A retention portion (322) is provided inside the column portion (318). The retention portion (322) serves to support a plunger driver (480), which provides moving force to the plunger (410). A column portion hole (324) is formed on the side of the column portion (318) to penetrate inside and out of the side of the column portion (318). A safety pin (490) may be inserted into the column portion hole (324) to restrict the movement of the plunger (410) and the carrier (422). Additionally, a column portion rail (325) and a fixing portion (326) are provided on the outer surface of the column portion (318). The column portion rail (325) is for guiding the carriage (610) of the removing unit (600), and the fixing portion (326) is for fixing the carriage driver (630) of the removing unit (600). In addition, an inner rail (327) and a detent portion (328) are provided inside the column portion (318). The inner rail (327) may guide the plunger (410) to induce stable linear movement of the plunger (410). The detent portion (328) is used to fix the plunger (410), which has moved from the first position to the second position, to the second position.

The specific configuration of the base frame (310) is not limited to that shown and can be changed in various ways.

The middle frame (330) includes an outer frame (331) coupled to the base frame (310), a stage (333) disposed inside the outer frame (331), and a support fixture (339) protruding from the stage (333). The outer frame (331) is provided with a hook portion (332) that can be engaged with the top case (370). The stage (333) is disposed on the frame base portion (311). A middle frame opening (334) into which the column portion (318) is inserted is provided in the middle of the stage (333). On both sides of the middle frame opening (334), a through hole (335) is formed into which the rod (363) of the locking hook (360) is inserted. The rod (363) may pass through the through hole (335) and protrude onto the stage (333). A guide protrusion (336) and an opening (337) are provided on the stage (333). The guide protrusion (336) serves to guide the moving member (500) to move linearly. The stopper arm (384) of the protective cap (380) may be inserted into the opening (337). An operating member (345) capable of operating the moving member (500) is installed on the support fixture (339).

The operating member (345) includes a button (346) and a pressing protrusion (347) protruding from the button (346). The operating member (345) can be operated by a user to move the moving member (500). When a user presses the button (346), the operating member (345) moves so that the pressing protrusion (347) pushes the moving member (500). The insertion unit (400) and the removing unit (600) may be operated in conjunction with the moving member (500) moved by the operating member (345). In addition to the configuration shown, the operating member (345) can be changed to various different configurations that are installed on the applicator body (300) so as to be operated by a user and may operate the insertion unit (400) and the removing unit (600).

A return member (350) is provided on one side of the middle frame (330). The return member (350) can be elastically deformed by the moving member (500) and serves to return the moving member (500) moved by the operating member (345) to its original state. The return member (350), which is elastically deformed by the moving member (500) moved forward by the operating member (345), can apply an elastic force to the moving member 500 in a retreating direction. In the drawing, the moving member (500) is shown as being arranged to face the support fixture (339) on one side of the stage (333). However, the location of the moving member (500) or its specific configuration is not limited to what is shown and can be changed in various ways.

The specific configuration of the middle frame (330) is not limited to that shown and can be changed in various ways. As another exemplary embodiment, the middle frame (330) may be formed integrally with the base frame (310).

The top case (370) is coupled with the middle frame (330) and covers the upper portions of the middle frame (330) and the base frame (310). On one side of the top case (370), a top case opening (371) is provided in a shape which allows the support fixture (339) of the middle frame (330) to be coupled. The top case (370) has a coupling hole (372) and can be coupled to the middle frame (330) by engaging the hook portion (332) of the middle frame (330) with the coupling hole (372).

The specific configuration of the top case (370) is not limited to that shown and can be changed in various ways. Additionally, the method of coupling the top case (370) and the middle frame (330) can also be changed in various ways.

The protective cap (380) is detachably coupled to the applicator body (300) so as to cover the bottom portion (312) of the applicator body (300). The protective cap (380) is coupled to the applicator body (300) and can cover and protect the base unit (200) mounted in the recess (313) of the applicator body (300). The protective cap (380) includes a cap body (381), an elastic arm (382), which is elastically deformable, provided on the cap body (381) and a stopper arm (384) for restraining the movement of the moving member (500). The elastic arm (382) is provided with a locking protrusion (383) inserted into the locking groove (340) provided on the outer frame (331). The protective cap (380) partially covers the middle frame (330) and is coupled to the middle frame (330). At this time, the locking protrusion (383) is inserted into the locking groove (340) of the outer frame (331), so that the protective cap (380) can remain stably coupled to the middle frame (330). The protective cap (380) can be easily separated from the middle frame (330) when a user pulls it with a certain amount of force or more.

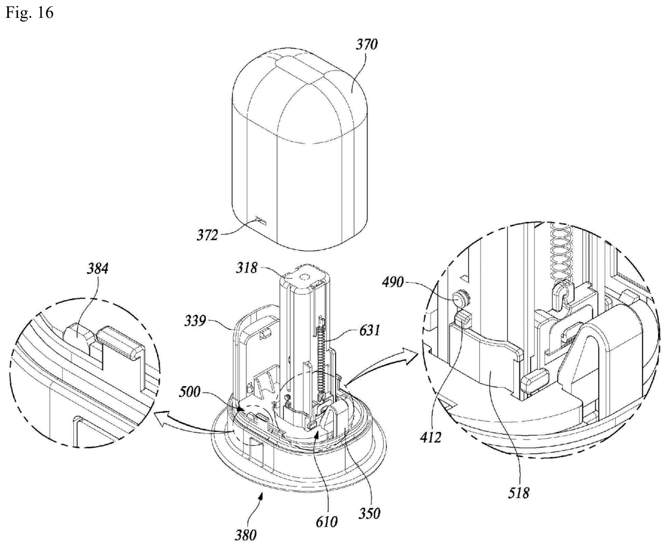

As shown in FIG. 16, when the protective cap (380) is coupled to the middle frame (330), the stopper arm (384) sequentially passes through the through hole (315) of the base frame (310) and the opening (337) of the middle frame (330) and engages with the moving member (500). While the stopper arm (384) is engaged with the moving member (500), the moving member (500) cannot move. Therefore, It is possible to prevent a problem in which the applicator (30) operates because the operating member (345) is manipulated due to a user's carelessness while storing or transporting the applicator assembly (10).

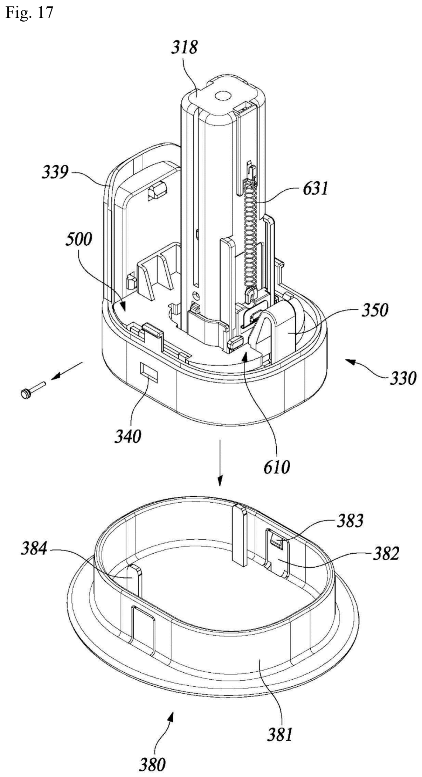

As shown in FIG. 17, the applicator (30) can be operated after the protective cap (380) is separated from the applicator body (300).

The insertion unit (400) is installed on the applicator body (300) to associate with the moving member (500) and move the sensor unit (100) from the first position to the second position and then insert the sensor (110) into the skin of a user. The insertion unit (400) includes the plunger (410) installed movably on the middle frame (330), a needle assembly (420) including a plunger (410) installed movably on the middle frame (330) and the needle (450) that can be moved with the plunger (410) and inserted into the skin of a user.

The plunger (410) is installed inside the column portion (318) to be movable from the first position to the second position. A plunger hook (411) is provided on one side of the plunger (410) to limit the movement range of the plunger (410). The plunger hook (411) protrudes from the plunger (410) and can limit the movement of the plunger (410) by contacting the detent portion (321) of the column portion (318) when the plunger (410) moves to the second position. A plunger protrusion (412) is provided on the other side of the plunger (410). The plunger protrusion (412) protrudes from the plunger (410) so as to engage with the moving member (500). The plunger (410) is fixed in the first position when the plunger protrusion (412) engages with the moving member (500), and can move to the second position when the plunger protrusion (412) is disengaged from the moving member (500).

A pair of slits (413) are formed parallel to the moving direction of the sensor unit (100) on both sides of the plunger (410). A detent portion (414) with which the latch portion (437) of the carrier (422) can be engaged is provided in the middle portion of the slit (413). In a state where the latch portion (437) is engaged with the detent portion (414), the carrier (422) moves together with the plunger (410) and cannot move relative to the plunger (410). A retention portion (415) and a fixing portion (416) are provided inside the plunger (410). The retention unit (415) supports the plunger driver (480). A needle release driver (485) that provides a moving force to the carrier (422) is coupled to the fixing portion (416). A plunger rail portion (417) and a plunger hole (418) are provided on the side of the plunger (410). The plunger rail portion (417) is configured to engage with the inner rail (327) of the column portion (318). The plunger (410) can stably move linearly along the inner rail (327) by engaging the plunger rail portion (417) with the inner rail (327). In the drawing, the inner rail (327) is shown to be in a protruding form, and the plunger rail portion (417) is shown to be in the form of a groove into which the inner rail (327) is inserted, but the configuration of the inner rail (327) and the plunger rail portion (417) can be changed in various ways. The plunger hole (418) is formed to penetrate the side of the plunger (410) inside and out. A safety pin (490) is inserted into the plunger hole (418). When the safety pin (490) passes through the column hole (324) and is inserted into the plunger hole (418), the movement of the plunger (410) may be restricted.

In addition, a plunger stopper (419) is provided on the outer surface of the plunger (410). The plunger stopper (419) may be engaged with the detent portion (328) of the column portion (318). When the plunger (410) moves to the second position, the plunger stopper (419) engages the detent portion (328) of the column portion (318), so that the plunger (410) can be fixed to the second position. After the applicator (30) operates to insert the sensor (110) into the skin of a user, the plunger (410) is fixed in the second position, thereby preventing reuse of the applicator (30). In addition, safety problems that may occur when the needle (450) is reloaded after use of the applicator (30) can be prevented. The configuration for fixing the plunger (410) in the second position is not limited to that shown and may vary in various ways.

The plunger (410) moves by receiving a moving force from the plunger driver (480). The plunger driver (480) includes an elastic member (481) that applies elastic force to the plunger (410) in a direction of moving from the first position to the second position. The elastic member (481) has one end in contact with the retention portion (322) of the column portion (318) and the other end in contact with the retention portion (415) of the plunger (410). The plunger (410) may be fixed in the first position by engaging the moving member (500) in a state in which the elastic member (481) is elastically deformed. The plunger driver (480) may be changed to various other configurations that can provide a moving force to the plunger (410) in addition to the configuration including the elastic member (481) in the form of a coil spring.

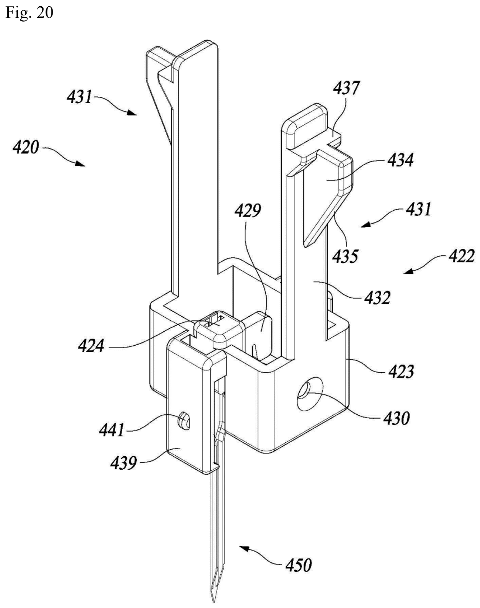

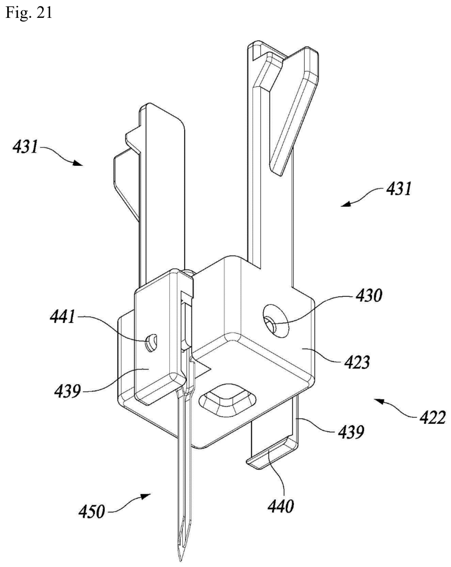

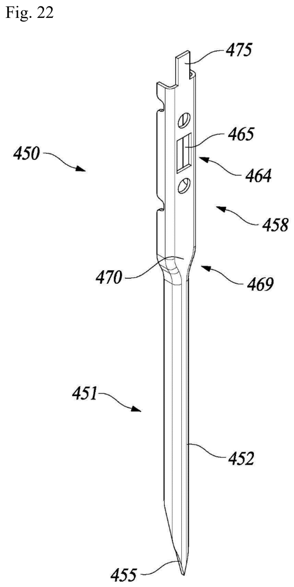

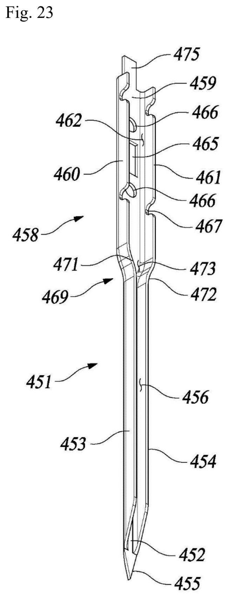

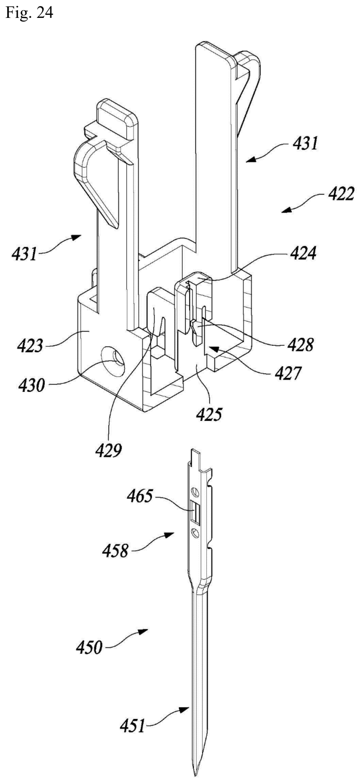

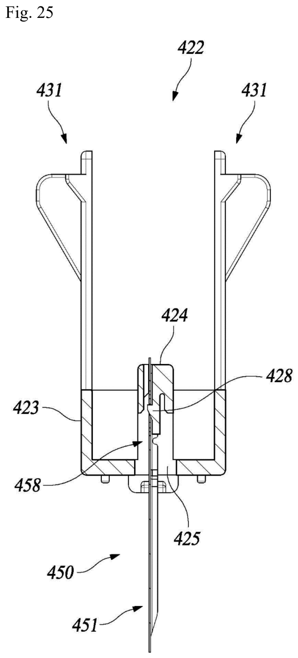

Referring to FIGS. 20 to 25, the needle assembly (420) includes the carrier (422) to which the sensor unit (100) is detachably coupled, and a needle (450) coupled to the carrier (422) penetrating the sensor unit (100). In a state in which the needle (450) is coupled to the sensor unit (100), the needle (450) advance from the first position to the second position with the sensor unit (100) to be inserted into the skin of a user, and then may retreat with the carrier (422) in the second position and be separated from the skin of a user and the sensor unit (100).

The carrier (422) is coupled to the plunger (410) to enable relative movement. A portion of the carrier (422) engages the plunger (410) so as to move with the plunger (410) from the first position to the second position. Then, the carrier (422) is disengaged from the plunger (410) in the second position and may retreat in a direction away from the sensor unit (100).

The carrier (422) includes a carrier body (423) to which the needle (450) is coupled, an elastically deformable carrier wing (431) extending from the carrier body (423), and a locking arm (439) connected to the side of the carrier body (423).

The carrier body (423) includes a boss (424) with an insertion groove (425) formed on the inside. A portion of the needle (450) may be inserted into the insertion groove (425). A clamp portion (427) for fixing the needle (450) is provided in the insertion groove (425). The clamp portion (427) includes a hook (428) that can engage with the needle (450). A fixing portion (429) to which the needle release driver (485) is coupled is provided on one side of the carrier body (423). The carrier (422) can be moved relative to the plunger (410) by the needle release driver (485).

The needle release driver (485) provides a moving force to the carrier (422). The needle release driver (485) includes an elastic member (486) whose one end is coupled to the fixing portion (416) of the plunger (410) and the other end is coupled to the fixing portion (429) of the carrier (422). The elastic member (486) is in the form of a tension spring and applies an elastic force to pull the carrier (422) toward the fixing portion (416) of the plunger (410). That is, the elastic member (486) can apply an elastic force to the carrier (422) in a direction away from the sensor unit (100) to move the needle (450) coupled to the carrier (422) to be separated from the sensor unit (100). The needle release driver (485) may be changed to various other configurations that can provide a moving force to the carrier (422) in addition to the configuration including the elastic member (486) in the form of a coil spring.

A carrier hole (430) is formed on the side of the carrier body (423) to penetrate the side of the carrier body (423) inside and out. The safety pin (490) may be inserted into the carrier hole (430). The safety pin (490) may fix the carrier (422) in the first position by inserting the carrier (422) into the carrier hole (430) while the carrier (422) is positioned in the first position.

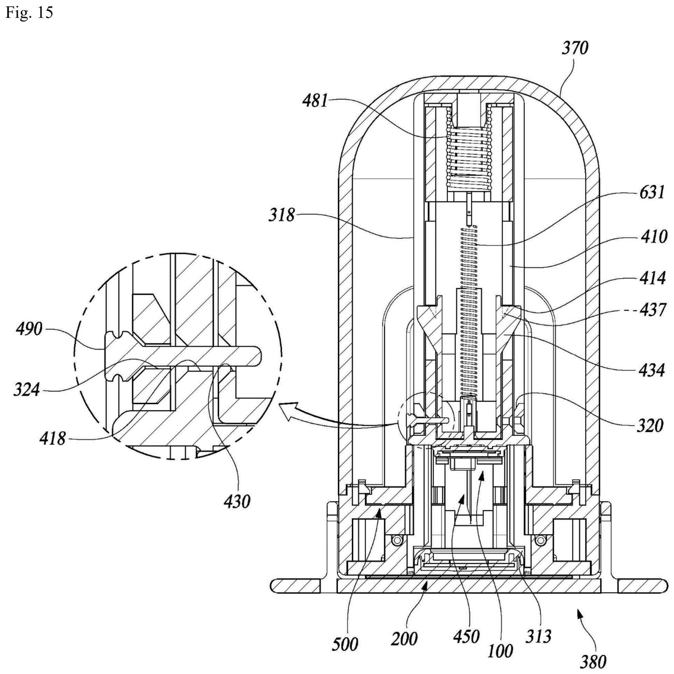

Shown as FIGS. 15 and 16, The safety pin (490) may be inserted into the column hole (324) of the column portion (318), the plunger hole (418) of the plunger (410), and the carrier hole (430) of the carrier( 422). When the plunger (410) is located in the first position, the column hole (324), the plunger hole (418), and the carrier hole (430) are aligned. In this state, the safety pin (490) can be inserted into the carrier hole (430) by sequentially passing through the column hole (324) and the plunger hole (418). At this time, the safety pin (490) may fix the plunger (410) and the carrier (422) in the first position. Therefore, the safety pin (490) can prevent malfunction of the applicator (30) due to the user's carelessness. As shown in FIG. 17, after separating the safety pin (490) from the column portion (318) , the user may operate the applicator (30) to insert the sensor into the skin.

The specific configuration and number of safety pins (490) may vary. As another exemplary embodiment, the safety pin passing through the column portion may be installed to engage the plunger without reaching the carrier. In this case, the safety pin can prevent malfunction of the applicator due to the user's carelessness by restraining the movement of the plunger in the first position.

The carrier wing (431) is connected to the carrier body (423) in a shape that extends from the carrier body (423) in the moving direction of the carrier (422). The carrier wing (431) includes a wing body (432) elastically deformably connected to the carrier body (423), and a trigger (434) and a latch portion (437) protruding from the wing body 432.

The trigger (434) is provided on one side of the wing body (432) to protrude outward from the wing body (432). The trigger (434) is inserted into the slit (413) of the plunger (410) and the slit (319) of the column portion (318) and can move along these slits (413) and (319). The trigger (434) is inserted into the slits (413 and 319) and guided by the slits (413 and 319), so that the carrier (422) can move linearly more stably. The trigger (434) passes through the slit (413) of the plunger (410), and some part is inserted into the slit (319) of the column portion (318). While the carrier (422) moves with the plunger (410) from the first position to the second position, the trigger (434) may contact the release portion (320) disposed in the middle of the slit (319) of the column portion (318).

As shown in FIG. 15, the release portion (320) is disposed in the middle of the slit (319) of the column portion (318) to press the trigger (434). While the carrier (422) is moving from the first position to the second position, the trigger (434) may come into contact with the release portion (320) and the carrier wing (431) may be elastically deformed. The trigger (434) is provided with a trigger inclined portion (435) that contacts the release portion (320). While the carrier (422) moves from the first position to the second position, the trigger inclined portion (435) comes into contact with the release portion (320), so that the carrier wing (431) can be elastically deformed more smoothly, and the impact that occurs when the trigger (434) comes into contact with the release portion (320) is reduced, and the pressing force of the release portion (320) can be more smoothly transmitted to the trigger (434).

The latch portion (437) protrudes outward from the wing body (432). The direction in which the latch portion (437) protrudes from the wing body (432) is the same as the direction in which the trigger (434) protrudes from the wing body (432). The protrusion height at which the latch portion (437) protrudes from the wing body (432) is lower than the protrusion height at which the trigger (434) protrudes from the wing body (432). The latch portion (437) may be engaged with the detent portion (414) of the plunger (410). In a state in which the latch portion (437) is engaged with the detent portion (414), the carrier (422) can maintain the elastic member (486) in an elastically deformed state, and cannot move in the direction in which the elastic force of the elastic member (486) acts.

The locking arm (439) is provided on the side of the carrier body (423) to be elastically deformable. A pair of locking arms (439) are arranged to face each other on both sides of the carrier body (423) and detachably fix the sensor unit housing (120) of the sensor unit (100). The locking arm (439) is provided with a locking protrusion (440) engaged with the sensor unit housing (120) and a protrusion (441) in contact with an inner wall (323) of the column portion (318). As shown in FIG. 14, when the carrier (422) and the sensor unit (100) are located in the first position, as the protrusion (441) contacts the inner wall (323) of the column portion (318), the locking arm (439) is biased in a direction in which it engages with the sensor unit housing (120). Accordingly, the sensor unit (100) can remain stably coupled to the carrier (422) in the first position.

Meanwhile, when the sensor unit (100) and the carrier (422) move toward the base unit (200) and the sensor unit (100) is coupled with the base unit (200), the protrusion (441) is located in a space connected to the recess (313) in which the base unit (200) is accommodated and deviates from the inner wall (323) of the column portion (318). Therefore, when the sensor unit (100) is coupled to the base unit (200), the fixing force of the locking arm (439) is weakened. And the sensor unit (100) is fixed to the base unit (200) by the adhesive layer (155), so that when the carrier (422) is pulled in a direction away from the body attachable unit (20) by the needle release driver (485), the locking arm (439) can be smoothly disengaged from the sensor unit (100) and separated from the sensor unit (100).

In addition to the configuration shown, the carrier 422 can be changed to various other configurations in which it is coupled to the needle (450) and installed to be relatively movable on the plunger (410). For example, the drawing shows a pair of carrier wings (431) being symmetrically provided on both sides of the carrier body (423), but the number and shape of the carrier wings (431) can be changed in various ways. Additionally, the coupling method of the carrier (422) and the needle (450) and the coupling method of the carrier (422) and the sensor unit (100) may also be changed in various ways.

The needle (450) is fixed to the carrier (422) and can move from the first position to the second position while coupled to the sensor unit (100). The needle (450) has a sharp tip so as to pierce the skin of a user and be smoothly inserted into the skin of a user. When the sensor unit (100) moves to the second position, the needle (450) penetrates the skin of a user before the sensor (110) and allows the sensor (110) to be stably inserted into the skin. The needle (450) is separated from the skin of a user after the sensor (110) is inserted into the skin of a user.

The needle (450) includes a needle body (451) inserted into the skin of a user, a needle head (458) coupled to the carrier (422), a needle neck (469) connecting the needle body (451) and the needle head (458), and a needle tail (475) extending from the end of the needle head (458). The needle (450) may be formed as one piece by press-processing the needle body (451), the needle head (458), the needle neck (469), and the needle tail (475) from the same base material.

The needle body (451) includes a needle body base (452) equipped with a needle tip (455) at the end, and a first needle body wing (453) and a second needle body wing (454) disposed on both sides of the needle body base (452). The first needle body wing (453) and the second needle body wing (454) are bent at both sides of the needle body base (452) and are arranged to face each other. The needle body base (452), the first needle body wing (453), and the second needle body wing (454) form the first channel (456). The insertion portion (116) of the sensor (110) may be inserted into the first channel (456). The needle (450) is coupled to the sensor unit (100) so that the insertion portion (116) is inserted into the first channel (456).

The needle head (458) includes a needle head base (459) disposed on the same plane as the needle body base (452), and a first needle head wing (460) and a second needle head wing (461) disposed on both sides of the needle head base (459). The first needle head wing (460) and the second needle head wing (461) are bent at both sides of the needle head base (459) and are arranged to face each other. The needle head base (459), the first needle head wing (460), and the second needle head wing (461) form the second channel (462). The width of the second channel (462) is larger than the width of the first channel (456).

The needle head (458) is provided with a locking portion (464) that engages the clamp portion (427) of the carrier (422). The locking portion (464) includes a needle hole (465) into which the hook (428) of the clamp portion (427) can be inserted. The needle hole (465) is provided in a form that penetrates the needle head base (459) in the thickness direction. The needle (450) may be fixed to the carrier (422) in such a way that the needle head (458) is inserted into the insertion groove (425) of the carrier (422) and the hook (428) is inserted into the needle hole (465).

In addition, the needle head (458) is provided with a through hole (466) and a groove portion (467). The through hole (466) is formed to penetrate the needle head base (459) in the thickness direction, and the groove portion (467) is formed in the first needle head wing (460) and the second needle head wing (461) respectively. The through hole (466) and the groove portion (467) may be used to reduce the weight of the needle (450) and prevent deformation of the needle head (458) in the process of forming the needle head (458) from the base material. Additionally, the through hole (466) and the groove portion (467) may be used for coupling with the carrier (422).

The needle neck (469) has a shape whose thickness gradually decreases from the needle head (458) toward the needle body (451) and connects the needle body (451) and the needle head (458). The needle neck (469) includes a needle neck base (470) disposed on the same plane as the needle body base (452), a first needle neck wing (471) and a second needle neck wing (472) disposed on both sides of the needle neck base (470). The first needle neck wing (471) and the second needle neck wing (472) are bent at both sides of the needle neck base (470) and are arranged to face each other. The needle neck base (470) connects the needle body base (452) and the needle head base (459), and the first needle neck wing (471) connects the first needle body wing (453) and the first needle head wing (460). And the second needle neck wing (472) connects the second needle body wing (454) and the second needle head wing (461). The needle neck base (470), the first needle neck wing (471), and the second needle neck wing (472) form the third channel (473). The third channel (473) has a shape whose width gradually decreases from the second channel (462) toward the first channel (456), and is connected to the first channel (456) and the second channel (462), respectively.

In the process of coupling the sensor (110) and the needle (450), the sensor (110) and the needle (450) are coupled by inserting the insertion portion (116) of the sensor (110) into the first channel (456) through the second channel (462) and the third channel (473) sequentially. In this way, since the needle (450) has a form in which the insertion portion (116) of the sensor (110) may be inserted into the first channel (456) through the second channel (462) and the third channel (473) which have relatively larger widths, a problem in which the sensor (110) is damaged or deformed when the sensor (110) and the needle (450) are coupled may be reduced.

The needle tail (475) extends from the end of the needle head base (459) to be disposed on the same plane as the needle head base (459). The width of the needle tail (475) is smaller than the width of the needle head base (459).

The needle (450) may be formed as one piece by press processing the needle body (451), the needle head (458), and the needle neck (469) from the same base material. This needle (450) has a simple structure, is easy to manufacture, and has a low manufacturing cost. And the needle (450) can be simply assembled to the carrier (422) by simply inserting the needle head (458) into the insertion groove (425) of the carrier (422). When the needle head (458) is inserted into the insertion groove (425) to a certain depth, the hook (428) of the carrier (422) is inserted into the needle hole (465) of the needle head (458), so that the needle (450) can be firmly fixed to the carrier (422).

The specific configuration of the needle (450) is not limited to that shown and may be changed in various ways.

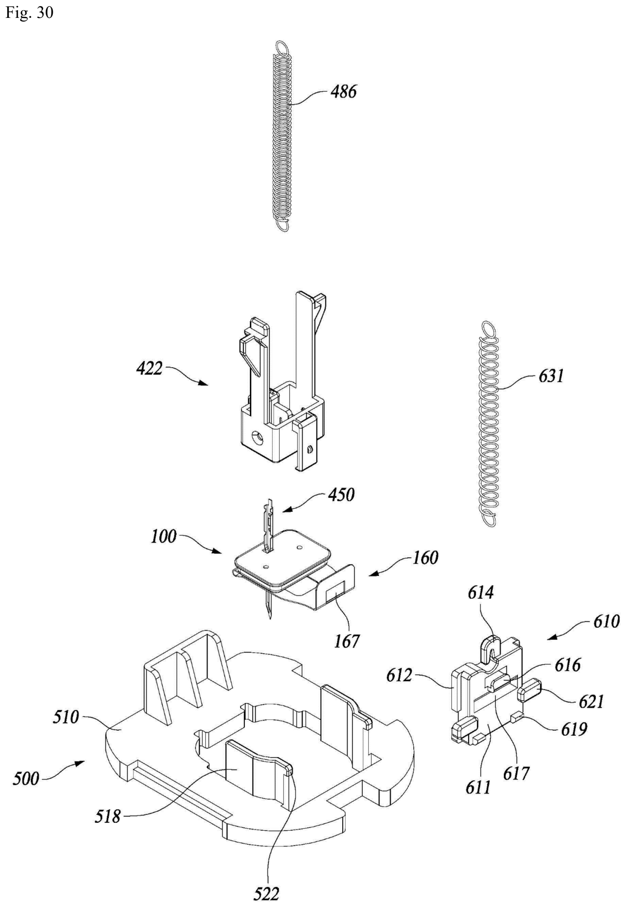

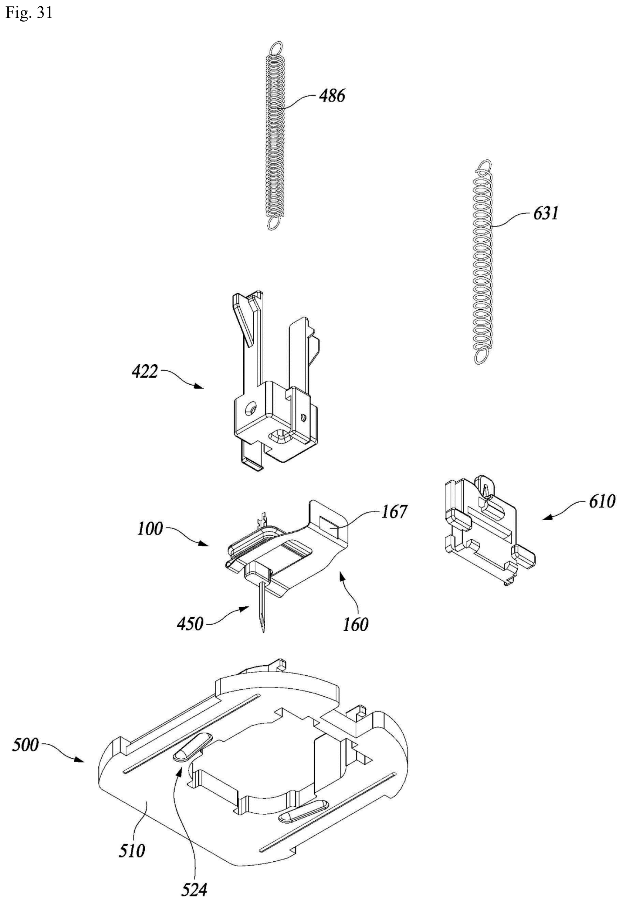

Referring to FIGS. 14 to 19 and 26 to 31, the moving member (500) is installed on the applicator body (300) to associate with the insertion unit (400) and the removing unit (600). The moving member (500) is arranged to move linearly on the stage (333) of the middle frame (330). The moving member (500) may move in a direction that is approximately perpendicular to the direction in which the sensor unit (100) moves from the first position to the second position. As a user manipulates the operating member (345), the moving member (500) advances toward the return member (350) and elastically deforms the return member (350), and then retreat toward the operating member (345) by the elastic force of the return member (350) and return to its original state. The moving member (500) includes a moving member body (510), a supporting portion (518) for supporting the plunger (410), and a first detent portion (520) and a second detent portion (522) which are capable of engaging with the carriage (610) of the removing unit (600).

A moving member opening (512) is formed in the middle of the moving member body (510) to penetrate the moving member body (510) in the thickness direction. A column portion (318) is inserted into the moving member opening (512). A bracket portion (513) is provided at one end of the moving member body (510) to protrude from the surface of the moving member body (510). The moving member (500) is disposed on the stage (333) so that the bracket portion (513) faces the support fixture (339) of the middle frame (330). The pressing protrusion (347) of the operating member (345) installed on the support fixture (339) may contact the bracket portion (513) to press the bracket portion (513) toward the return member (350). Rail portions (515) are provided on both edges of the moving member body (510). The rail portion (515) may engage with the guide protrusion (336) of the middle frame (330). The moving member (500) can be coupled to the middle frame (330) so that the rail portion (515) does not deviate from the stage (333) by engaging the guide protrusion (336), and can stably move linearly on the stage (333). The connecting method the moving member body (510) and the middle frame (330) or the connecting method of the moving member body (510) and the operating member (345) is not limited to that shown and can be changed in various ways.

A pair of supporting portions (518) are disposed at both edges of the moving member opening (512) to face each other. The supporting portion (518) may protrude from the moving member body (510) and support the plunger protrusion (412) of the plunger (410). Before the moving member (500) is moved by the operating member (345), the supporting portion (518) may support the plunger protrusion (412) of the plunger (410) to fix the plunger (410) in the first position. And, when the moving member (500) advances toward the return member (350) by the operating member (345), the supporting portion (518) deviates from the plunger protrusion (412), thereby allowing the plunger (410) to move to the second position. The supporting portion (518) may be changed into various other shapes that can support the plunger protrusion (412) or a portion of the plunger (410) in addition to the shape that protrudes from the moving member body (510).

The first detent portion (520) is provided at the edge of the moving member opening (512) so as to engage with the carriage (610). When the carriage (610) engages with the first detent portion (520), the movement of the carriage (610) is restricted. The second detent portion (522) is located at a certain distance from the first detent portion (520) in the moving direction of the carriage (610) so as to engage with the carriage (610). As shown, the second detent portion (522) may be disposed at the edge of the supporting portion (518). When the carriage (610) engages with the second detent portion (522), the movement of the carriage (610) is restricted.

The moving member (500) can stand by at a position approaching the support fixture (339) in contact with the return member (350) when no other external force acts. In this state, the moving member (500) may engage the plunger (410) and fix the plunger (410) in the first position. And before the moving member (500) advances toward the return member (350) by the operating member (345), the carriage (610) is engaged with the first detent portion (520), and the carriage (610) can be fixed in a coupled state with the protective sheet (160) of the sensor unit (100) located in the first position. Meanwhile, when the moving member (500) moves forward toward the return member (350) by the operating member (345) and then moves backward by the elastic force of the return member (350), the carriage (610) can move away from the moving member (500).

In this way, the moving member (500) can operate the insertion unit (400) and the removing unit (600) in connection with the operating member (345). In addition to these functions, the moving member (500) can move the locking hook (360) that secures the base unit (200). For this purpose, a guide portion (524) engaged with a locking hook (360) is provided on one side of the moving member body (510) which is facing the stage (333) of the middle frame (330).

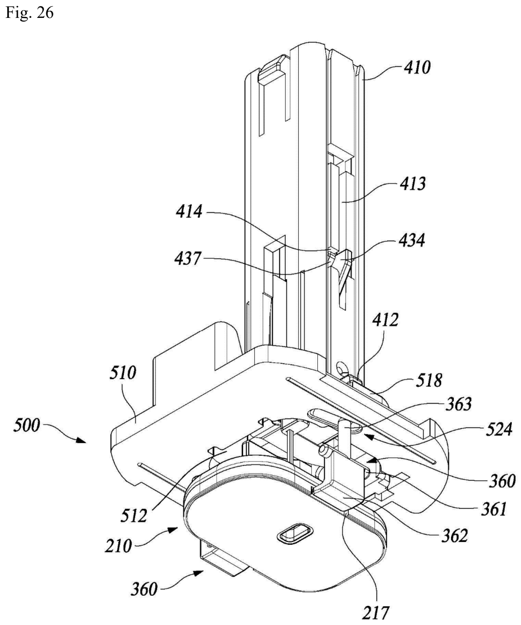

The guide portion (524) is formed in the form of a groove arranged to be inclined with respect to the moving direction of the moving member (500) so that the end of the rod (363) of the locking hook (360) can be engaged. Since the end of the rod (363) is engaged with the guide portion (524), the locking hook (360) can rotate in conjunction with the moving member 500. Shown in FIGS. 26 to 29, depending on the position of the moving member (500), the locking hook (360) may rotate in a direction in which the hook portion (362) engages with the base unit (200), or may rotate in a direction in which the hook portion (362) deviates from the base unit (200).

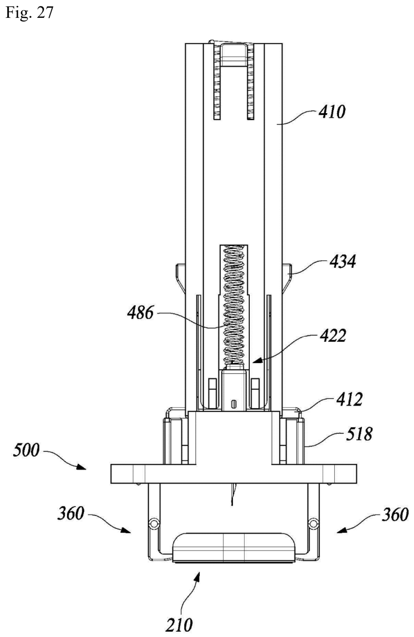

FIGS. 26 and 27, before the moving member (500) advances toward the return member (350) by the operating member (345), the guide portion (524) biases the locking hook (360) in a direction in which the hook portion (362) engages with the base unit (200).

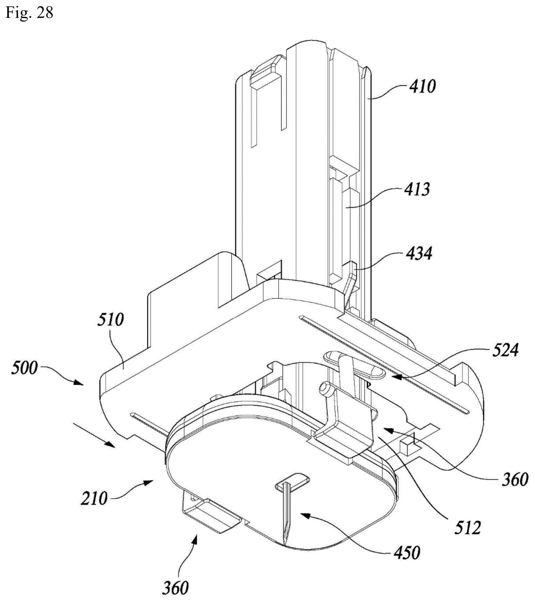

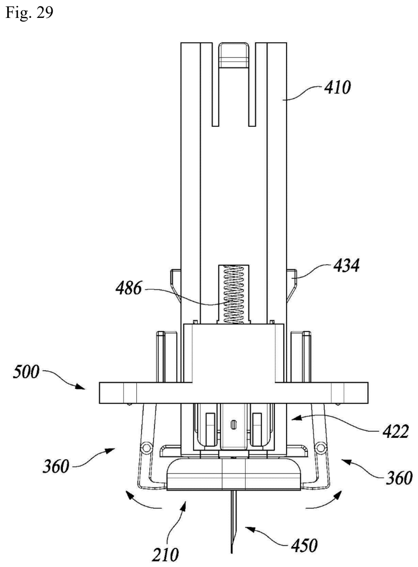

On the other hand, as shown in FIGS. 28 and 29, when the moving member (500) advances toward the return member (350), the guide portion (524) rotates the locking hook (360) in a direction in which the hook portion (362) is disengaged from the base unit (200). That is, when the moving member (500) moves forward toward the return member (350) by the operating member (345), the end of the rod (363) of the locking hook (360) is pulled by the guide portion (524) in a direction approaching the moving member opening (512) of the moving member (500). At this time, by rotating around the pivot portion (361), the locking hook (360) is biased so that the hook portion (362) is disengaged from the base unit (200).

Due to the action of the moving member (500), before the moving member (500) is moved by the operating member (345), the locking hook (360) maintains a state of being stably engaged with the base unit (200) so that the base unit (200) cannot be separated from the recess (313) of the applicator body (300). And when the moving member (500) moves forward to release the restraint on the plunger (410), the locking hook (360) may be disengaged from the base unit (200), and the base unit (200) can be separated from the applicator body (300). Therefore, after the sensor unit (100) is coupled with the base unit (200) and the body attachable unit (20) is assembled, the body attachable unit (20) can be smoothly separated from the applicator (30) while being attached to the skin of a user by the adhesive portion (230).

In the drawing, the guide portion (524) is shown as having a groove shape, but the guide portion is changed into various other shapes that engage with the locking hook (360) and can bias the locking hook (360) according to the movement of the moving member (500). Depending on the shape of the guide portion, etc., the locking hook may also be changed into various configurations that can be detachably engaged with the base unit (200).

Shown as FIGS. 14 to 19, 30, and 31, the removing unit (600) is installed on the applicator body (300) to separate the protective sheet (160) of the sensor unit (100) from the adhesive layer (155) before the sensor unit (100) reaches the second position and is coupled to the base unit (200). The removing unit (600) includes a carriage (610) installed movably on the applicator body (300) to be coupled to the protective sheet 160, and a carriage driver (630) that moves the carriage (610) by providing a moving force to the carriage (610).

The carriage driver (630) includes an elastic member (631) that applies elastic force to the carriage (610) in a direction away from the bottom portion (312) of the applicator body (300). The elastic member (631) has one end connected to the fixing portion (326) of the column portion (318) and the other end connected to the carriage (610). The carriage driver (630) may be changed into various other forms that can provide a moving force to the carriage (610) in addition to including the elastic member (631) in the form of a coil spring.

The carriage (610) may pull the protective sheet (160) to be separated from the adhesive layer (155) by moving while being coupled with the protective sheet (160). The carriage (610) is slidably installed on the outer surface of the column portion (318) and can move in a direction away from the sensor unit (100).

The carriage (610) includes a carriage body (611) and a detent protrusion (619) provided on one side of the carriage body (611) so as to engage with the first detent portion (520) of the moving member (500) and a stopper protrusion (621) provided on the other side of the carriage body (611) so as to engage with the second detent portion (522) of the moving member (500).

Carriage wings (612) engaged with the column rails (325) of the column portion (318) are provided on both edges of the carriage body (611). The carriage (610) can stably move linearly along the column rail (325) by engaging the carriage wing (612) with the column rail (325). The carriage (610) is guided by the column rail (325) and can move in a direction parallel to the moving direction of the plunger (410). A fixing portion (614) to which an elastic member (631) is connected is provided on one side of the carriage body (611). Additionally, among both surfaces of the carriage body (611), the surface that does not face the column portion (318) is provided with a grip portion (616) to which the wing portion (164) of the protective sheet (160) can be coupled. And in the middle part of the carriage body (611), a carriage hole (617) through which the wing portion (164) can pass is formed to penetrate the carriage body (611). The protective sheet (160) of the sensor unit (100) placed inside the column portion (318) may pass through the opening (329) of the column portion (318) and the carriage hole (617) and be coupled to the grip portion (616). The grip portion (616) may be coupled to the protective sheet (160) in such a way that a portion of the grip portion (616) is inserted into the protective sheet groove (165) of the wing portion (164).

The coupling method of the moving member (500) and the protective sheet (160) may be changed to various other methods other than using the grip portion (616). As another exemplary embodiment, the moving member (500) may be coupled to the protective sheet (160) through an adhesive method, or may be coupled to the protective sheet (160) by engaging an edge of the protective sheet (160).

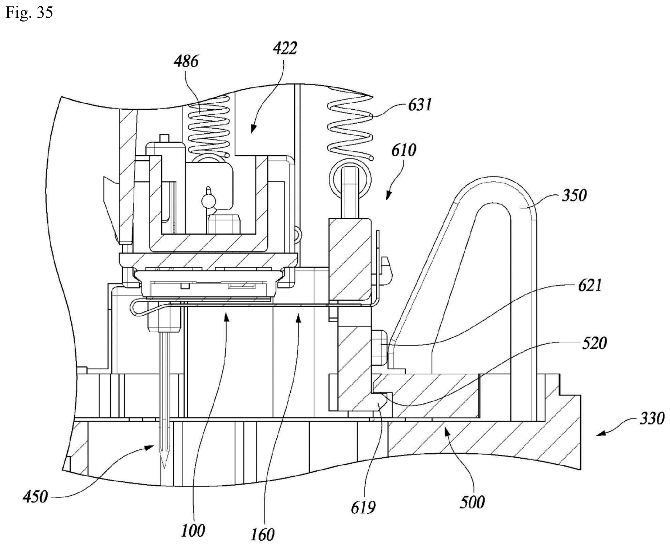

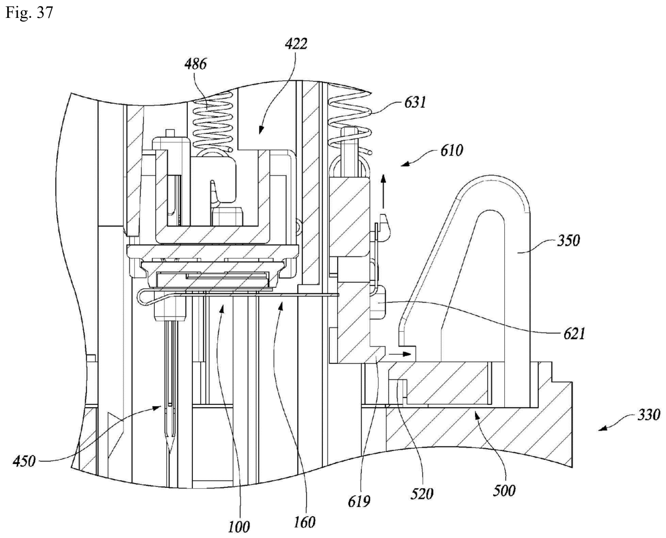

The detent protrusion (619) may protrude from one side of the carriage body (611) and engage with the first detent portion (520) of the moving member (500). As shown in FIG. 35, before the moving member (500) is moved by the operating member (345), the detent protrusion (619) engages with the first detent portion (520) and, at this time, the carriage (610) may be fixed in a state coupled to the protective sheet (160) while the sensor unit (100) is placed in the first position. Meanwhile, as shown in FIG. 37, when the moving member (500) advances a certain distance by the operating member (345), the detent protrusion (619) is disengaged from the first detent portion (520), and at this time, the carriage (610) can be moved a certain distance by the carriage driver (630).

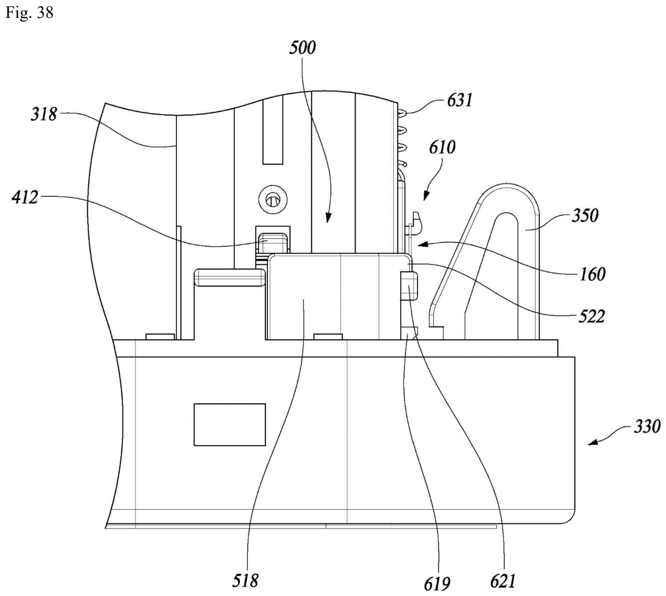

The stopper protrusion (621) protrudes from the other side of the carriage body (611) to engage with the second detent portion (522) of the moving member (500). As shown in FIG. 35, before the moving member (500) is moved by the operating member (345), the stopper protrusion (621) maintains a certain distance away from the second detent portion (522). Meanwhile, as shown in FIG. 38, when the moving member (500) advances a certain distance by the operating member (345) and the carriage (610) moves a certain distance by the carriage driver (630), the stopper protrusion (621) engages with the second detent portion (522). At this time, the carriage (610) cannot move, and the stopper protrusion (621) engages with the second detent portion (522), so the moving member (500) cannot move further forward. When the stopper protrusion (621) engages with the second detent portion (522) and the movement of the moving member (500) is restricted, the supporting portion (518) of the moving member (500) maintains the state of supporting the plunger (410). Therefore, even if a user presses the operating member (345) before the carriage (610) is completely removed from the moving member (500), the moving member (500) cannot advance to the position where the engagement with the plunger (410) is released, and the plunger (410) remains waiting at the first position.

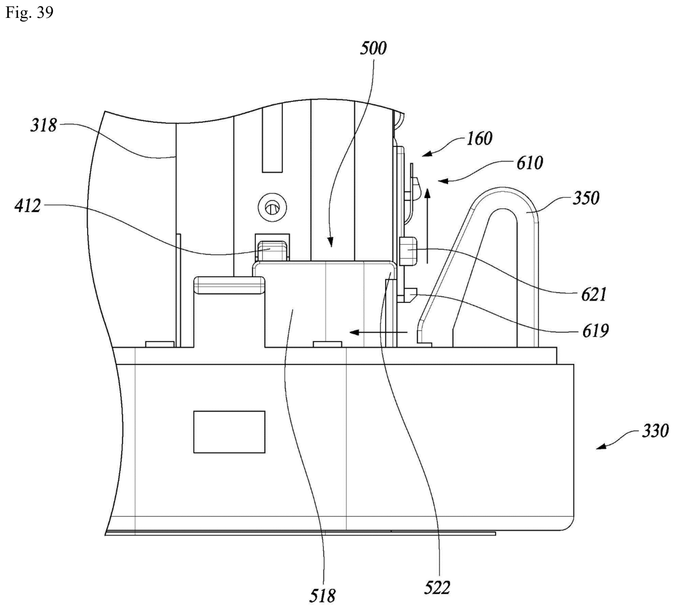

Meanwhile, when the force of a user pressing the operating member (345) is removed, the moving member (500) is retracted to the initial position by the return member (350), as shown in FIG. 39. At this time, the carriage (610) may drag the protective sheet (160) and move away from the bottom portion (312) to separate the protective sheet (160) from the adhesive layer (155).

In this way, the removing unit (600) may operate to complete the separation of the protective sheet (160) before the sensor unit (100) moves from the first position. Specifically, when the moving member (500) advances a certain distance by the operating member (345), the carriage (610) of the removing unit (600) moves a certain distance from the third position where the detent protrusion (619) engages with the first detent portion (520) of the moving member (500) to the fourth position where the stopper protrusion (621) engages with the second detent portion (522) of the moving member (500). The carriage (610) located at the fourth position restrains the movement of the moving member (500) so that the moving member (500) maintains the engaged state in the plunger (410). Then, the carriage (610) is released from engagement with the moving member (500) as the moving member (500) retreats to its original position by the return member (350), and the separation of the protective sheet (160) is completed by moving from the fourth position to the fifth position.

Therefore, when a user presses the operating member (345) once, only the removing unit (600) operates first in a state in which the insertion unit (400) does not operate, so the protective sheet (160) can be separated from the adhesive layer (155). And after the removing unit (600) operates, the moving member (500) can advance to a position where the engagement is released from the plunger (410) without interfering with the carriage (610). Therefore, when a user presses the operating member (345) once again, the moving member (500) moves to a position where the engagement is released from the plunger (410), so that the insertion unit (400) can operate.

If the sensor unit (100) is moved to the second position without the protective sheet (160) completely separated, a problem may occur in which the protective sheet (160) is caught between the sensor unit (100) and the base unit (200) or the sensor unit (100) is coupled to the base unit (200) without the protective sheet (160) being completely separated. In contrast, the applicator (30) according to the present embodiment completes the separation operation of the protective sheet (160) before the sensor unit (100) moves from the first position, so it can prevent the sensor unit (100) from reaching the base unit (200) without the protective sheet (160) being separated. This operation can be implemented through a configuration in which the first moving distance of the moving member (500) moving forward in order to disengage the detent protrusion (619) of the carriage (610) from the first detent portion (520) of the moving member (500) is shorter than the second moving distance of the moving member (500) moving forward in order to disengage the plunger (420) from the moving member (500). For this, the supporting portion (518) of the moving member (500) that supports the plunger (410) may be designed to extend an appropriate length in the moving direction of the moving member (500).

As the supporting portion (518) is designed to have different lengths extending in the direction of movement of the moving member (500), it is also possible that the movement of the sensor unit (100) toward the second position and the action of separating the protective sheet (160) proceed simultaneously.

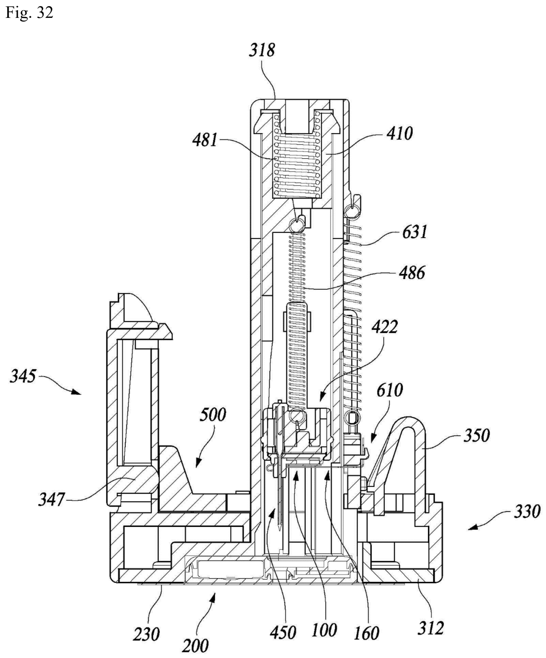

Hereinafter, with reference to FIGS. 32 to 44, a specific process of attaching the body attachable unit (20) to the skin of a user using the applicator (30) according to an embodiment of the present disclosure will be described.

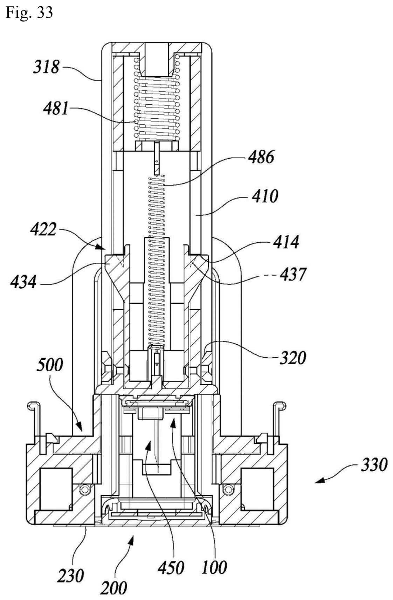

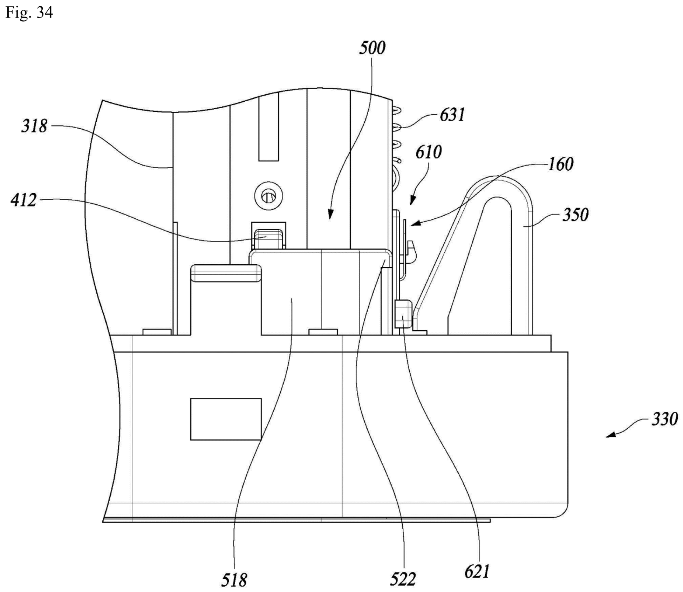

With reference to FIGS. 32 and 33, after the protective cap (380) is separated from the applicator body (300) and the safety pin (490) is removed, then the bottom portion (312) of the applicator (30) is positioned on the skin of a user so that the base unit (200) is attached to the skin of a user through the adhesive portion (230). Before the operating member (345) is manipulated, the sensor unit (100) coupled to the carrier (422) is positioned in the first position in a state in which the protective sheet (160) covers the adhesive layer (155). At this time, as shown in FIG. 34, the supporting portion (518) of the moving member (500) supports the plunger (410) and the plunger (410) is fixed in the first position. And as shown in FIG. 35, the detent protrusion (619) of the carriage (610) is engaged with the first detent portion (520) of the moving member (500), and the carriage (610) remains in the third position while being coupled with the protective sheet (160).

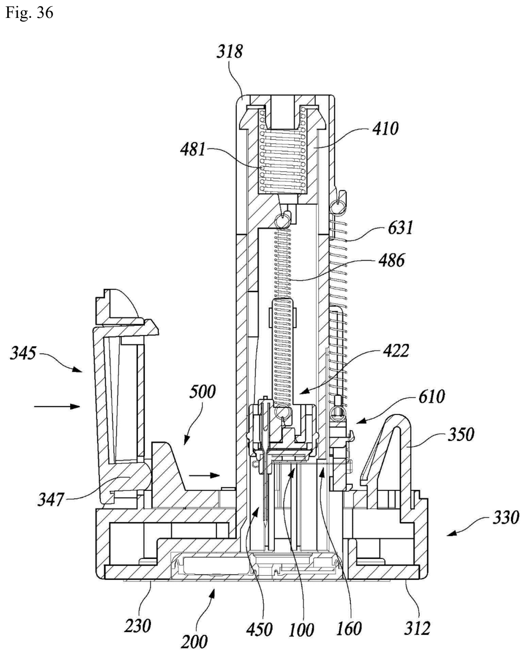

Thereafter, as shown in FIG. 36, when the operating member (345) is pressed, the operating member (345) presses the moving member (500), causing the moving member (500) to move forward while elastically deforming the return member (350). As the moving member (500) advances a certain distance by the operating member (345), the detent protrusion (619) is disengaged from the first detent portion (520), as shown in FIG. 37 . At this time, the carriage (610) moves a certain distance in a direction away from the bottom portion (312) of the applicator body (300) by the elastic force of the elastic member (631), and as shown in FIG. 38, the stopper protrusion (621) stops at the fourth position as it engages with the second detent portion (522) of the moving member (500). As the stopper protrusion (621) engages with the second detent portion (522), the moving member (500) cannot advance further, and the supporting portion (518) of the moving member (500) maintains the plunger (410) in a supported state.

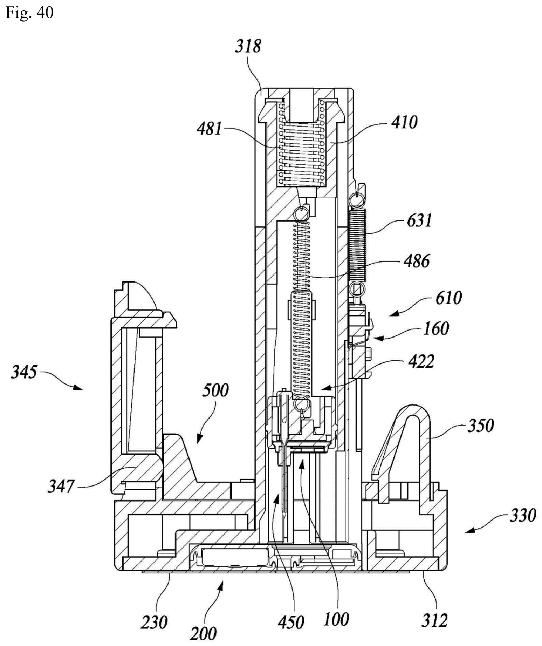

Meanwhile, when the force a user is pressing the operating member (345) is removed, as shown in FIG. 39, the moving member (500) retreats to the initial position by the return member (350), and the stopper protrusion (621) deviates from the second detent portion (522). Therefore, as shown in FIG. 40, the carriage (610) drags the protective sheet (160) by the elastic force of the elastic member (631) and moves to the fifth position to separate the protective sheet (160) from the adhesive layer (155).

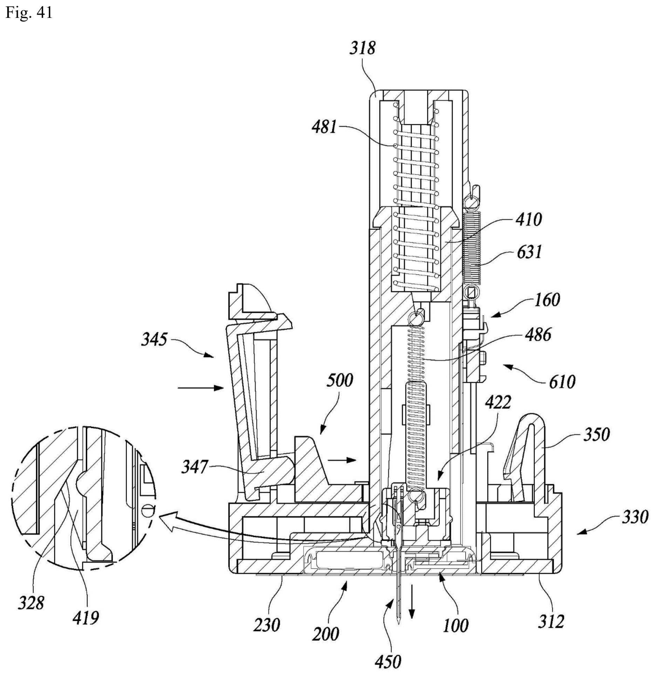

Thereafter, as shown in FIG. 41, when the operating member (345) is pressed again, the moving member (500) can advance to a position where the engagement with the plunger (410) is released. When the plunger (410) moves away from the moving member (500), the plunger (410) moves to the second position by the elastic force of the elastic member (481). At this time, the plunger (410) is fixed in the second position by engaging the plunger stopper (419) with the detent portion (328) of the column portion (318). Then, the insertion portion (116) of the sensor (110) and the needle (450) are inserted into the skin of a user, and the sensor unit (100) from which the protective sheet (160) is separated is attached to the base unit (200) by the adhesive layer (155), so that the body attachable unit (20) is assembled.

As previously described, the adhesive layer (155) couples the sensor unit (100) and the base unit (200) and seals between the sensor unit-electrical contact portion (146) of the sensor unit (100) and the base unit-electrical contact portion (225) of the base unit 200. In the process in which the body attachable unit (20) is attached to the skin of a user and measures biosignals, as the adhesive layer (155) seals the electrical connection portion (250) between the sensor unit (100) and the base unit (200), moisture, foreign substances, body fluids, etc. do not flow into the electrical connection portion (250) between the sensor unit (100) and the base unit (200).

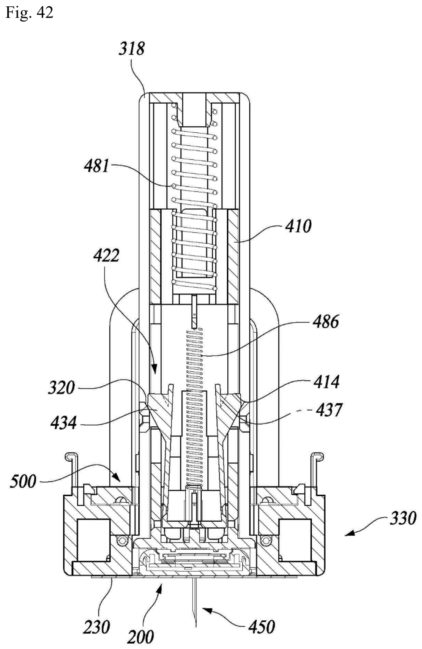

As shown in FIG. 42, when the plunger (410) moves and the insertion portion (116) and the needle (450) are inserted into the skin of a user, the trigger (434) of the carrier (422) comes into contact with the release portion (320) of the column portion (318). At this time, the carrier wing (431) of the carrier (422) is elastically deformed, so that the latch portion (437) of the carrier (422) is disengaged from the detent portion (414) of the plunger (410).

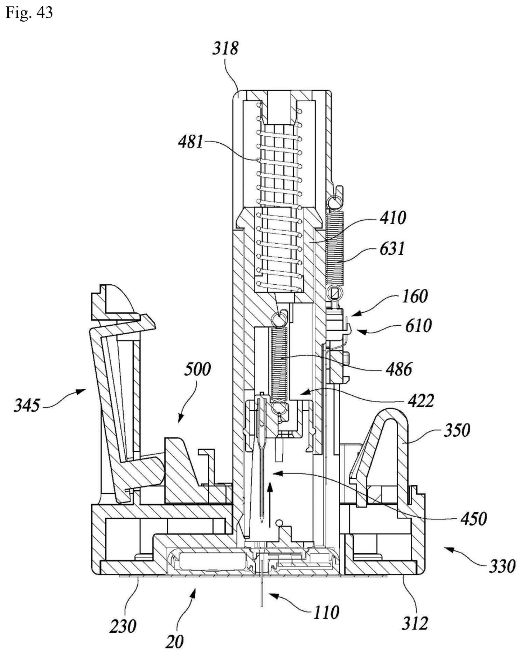

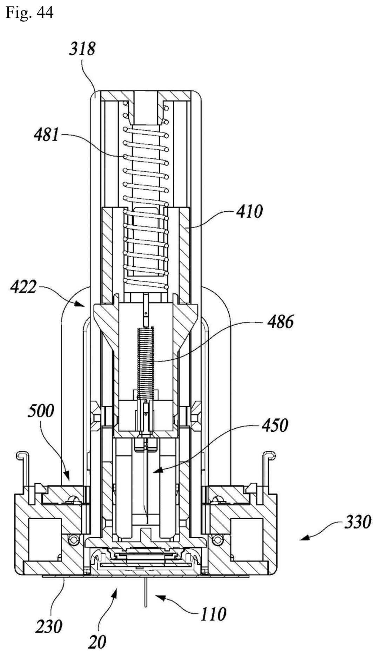

When the carrier (422) is disengaged from the plunger (410), the carrier (422) is moved away from the skin of a user by the elastic member (486), as shown in FIGS. 43 and 44. At this time, the needle (450) comes out of the skin of a user and the body attachable unit (20) remains attached to the skin of a user by the adhesive portion (230).

As described above, in the process of the needle (450) coming out of the skin of a user after the insertion portion (116) of the sensor (110) is inserted into the skin, the retention protrusion (136) of the sensor unit housing (120) comes into contact with the middle portion (113) of the sensor (110) and restricts the movement of the insertion portion (116). Accordingly, the insertion portion (116) cannot retreat in the same direction as the needle (450) and can remain stably inserted into the skin.

In a state in which the sensor unit (100) is coupled with the base unit (200) and the body attachable unit (20) is assembled, the locking hook (360) engaged with the base unit (200) rotates in conjunction with the moving member (500). That is, the locking hook (360) is biased to be disengaged from the base unit (200) by the moving member (500). Accordingly, the body attachable unit (20) can be separated from the applicator (30) while being attached to the skin of a user.

The body attachable unit (20) attached to the skin of a user and separated from the applicator (30) can measure the biometric information and transmit the measurement information to an external terminal (5) and the like.

As described above, the applicator assembly (10) according to an embodiment of the present disclosure is mounted on the applicator (30) with the adhesive layer (155) of the sensor unit (100) covered with the protective sheet (160), so that the adhesiveness of the adhesive layer (155) does not deteriorate even if it is not used for a long time. Then, as a user manipulates the operating member (345), the removing unit (600) operates to automatically remove the protective sheet (160) from the sensor unit (100), and the insertion unit (400) operates so that the sensor unit (100) is firmly coupled to the base unit (200) by the adhesive layer (155) and the sensor (110) is inserted into the skin of a user. Therefore, it is convenient to use, and the body attachable unit (20) can be more stably attached to the skin of a user.

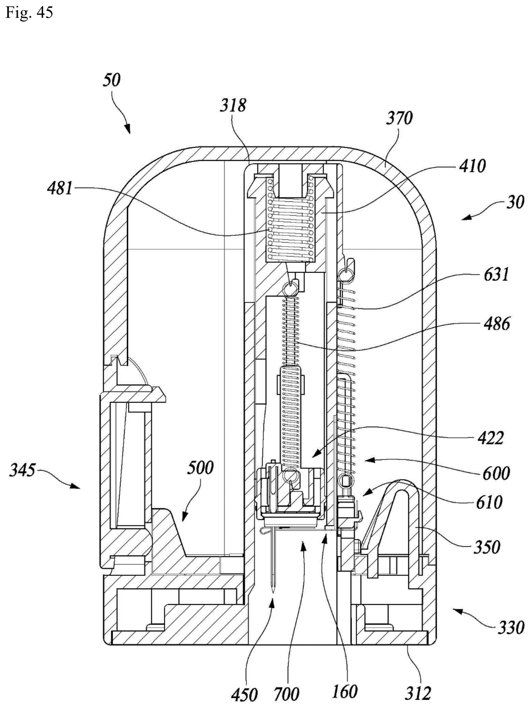

Meanwhile, FIG. 45 shows the applicator assembly according to another embodiment of the present disclosure.

Shown in FIG. 45, the applicator assembly (50) includes a sensor unit (700) having a sensor that is inserted into the skin of a user and measures biometric information, and the applicator (30) on which the sensor unit (700) is detachably mounted to insert the sensor into the skin of a user. The applicator (30) is the same as described previously.

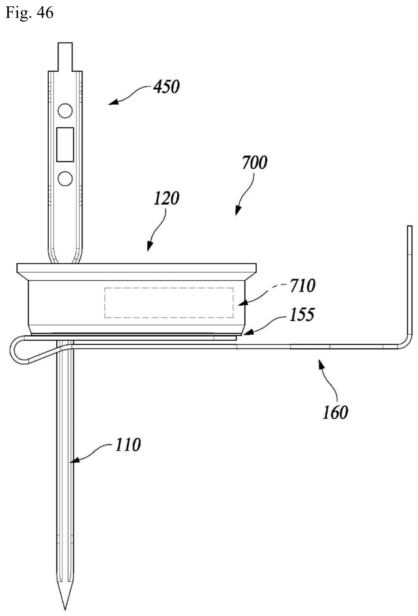

As shown in FIG. 46, the sensor unit (700) includes the sensor (110) inserted into the skin of a user, the sensor unit housing (120) to which the sensor (110) is coupled, the adhesive layer (155) provided on the sensor unit housing (120), the protective sheet (160) covering the adhesive layer (155), and an electronic component (710). The electronic component (710) includes a board electrically connected to the sensor (110), a process chip installed on the board to convert biometric information measured by the sensor (110) into an electrical signal, a communication chip for communication with the outside and batteries, etc. The sensor unit (700) can be attached to the skin of a user by moving from the first position to the second position while being coupled with the needle (450).

The applicator (30) may use the removing unit (600) to remove the protective sheet (160) from the sensor unit (700) and move the sensor unit (700) from the first position to the second position.

The sensor (110) is inserted into the skin of a user in a second position and the sensor unit (700) may be attached to the skin of a user by the adhesive layer (155). After the sensor unit (700) is attached to the skin of a user, the needle (450) is separated from the skin, and the sensor unit (700) is separated from the applicator (30) to measure the biometric information of a user.

Since the applicator assembly (50) according to this embodiment has a sensor unit (700) equipped with a signal processing function and a communication function, the sensor unit (700) is attached to the skin of a user and measures biometric information without the need for a separate base unit. The measured biometric information can be transmitted to an external terminal (5) or the like.

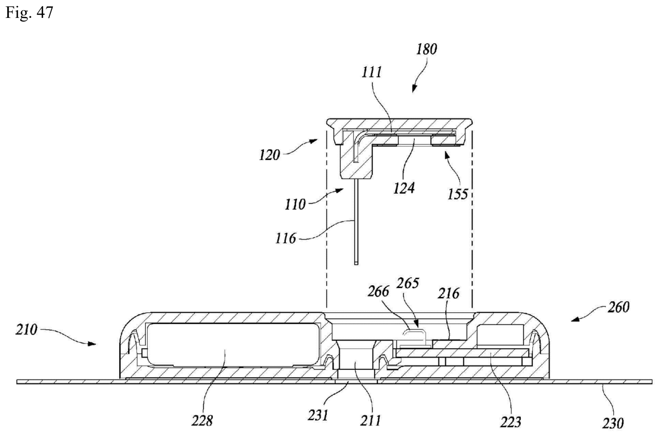

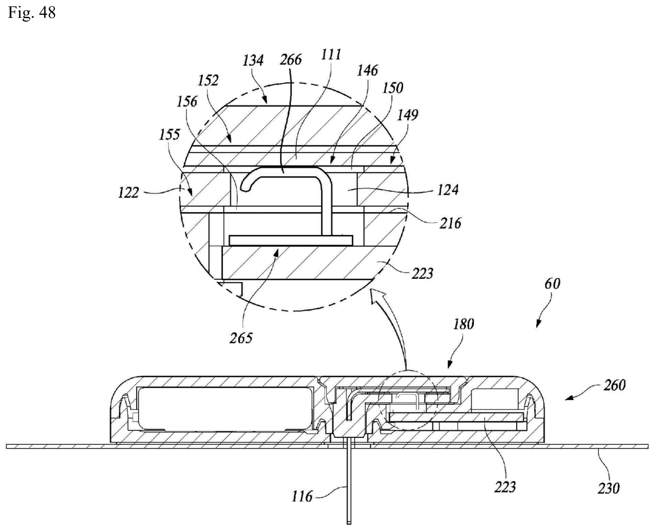

Meanwhile, FIGS. 47 and 48 show other embodiments of the sensor unit and base unit.

The sensor unit (180) shown in FIGS. 47 and 48 includes the sensor (110) inserted into the skin of a user, the sensor unit housing (120) to which the sensor (110) is coupled, the sensor adhesive portion (149) and an adhesive pad (152) for fixing to the sensor unit housing (120), the adhesive layer (155) provided on the sensor unit housing (120) to be attached to the base unit (260), and the protective sheet covering the adhesive layer (155). The sensor unit (180) has a configuration in which the sensor unit-electrical contact portion for electrically connecting the sensor (110) to the base unit (260) is omitted.

The base unit (260) includes the base unit housing (210) to which the sensor unit (180) is coupled, the circuit board (223) installed inside the base unit housing (210), the base unit-electrical contact portion (265) electrically connected to the sensor (110) of the sensor unit (180) and a battery (228). Base unit-electrical contact portion (225) contact sensor (110) when sensor unit (180) is coupled to base unit (260). The base unit-electrical contact portion (225) is electrically connected to the circuit board (223), and a portion of the base unit-electrical contact portion (225) may protrude from the contact surface (216) to contact the sensor (110). The base unit-electrical contact portion (225) may include a plurality of terminal portions (266) that each contact a plurality of contact points (not shown) provided on the sensor body (111) of the sensor (110). The terminal portion (266) may electrically connect the sensor (110) and the circuit board (223) by contacting a contact point of the sensor body (111). The terminal portion (266) may be formed to be elastically deformed when in contact with the sensor body (111) so as to stably contact the sensor body (111).

The sensor unit (180) is attached to the base unit (260) by the adhesive layer (155), thereby forming a body attachable unit (60) together with the base unit (260). When the sensor unit (180) is coupled with the base unit (260), the base unit-electrical contact portion (265) passes through the adhesive layer hole (156), the through hole (124), and the sensor adhesive hole opening (150) and comes into contact with the sensor body (111) of the sensor (110). Accordingly, the sensor (110) may be electrically connected to the base unit (260) through the base unit-electrical contact portion (265). At this time, the adhesive layer (155) seals between the sensor (110) and the base unit-electrical contact portion (265) to prevent moisture or foreign substances from entering the electrical connection portion between the sensor unit (180) and the base unit (260).

In order to electrically connect the sensor (110) and the base unit (260), the specific configuration, number, and location of the base unit-electrical contacts provided in the base unit (260) may be changed in various ways.

Although the present disclosure has been described above with preferred examples, the scope of the present disclosure is not limited to the form described and shown above.

For example, the drawing shows that the carriage having a grip portion to which a part of the protective sheet is coupled moves in a direction away from the sensor unit housing of the sensor unit to separate the protective sheet, but the method of separating the protective sheet can be changed in various ways. That is, the applicator according to the present disclosure can separate the protective sheet through various methods of changing the distance between the grip portion installed on the applicator body and the sensor unit housing. As another exemplary embodiment, the grip portion may be fixedly installed on one side of the applicator body to be spaced apart from the bottom of the applicator body. In this case, when the sensor unit is moved by the insertion unit, the protective sheet may be pulled by the grip portion and separated from the sensor unit housing. As another exemplary embodiment, the grip portion may be installed on the applicator body so that it can move in a direction away from the unit housing.

Additionally, the drawing shows that the carriage for separating the protective sheet from the sensor unit is installed to move opposite to the direction of movement of the sensor unit, but the carriage may be installed to move in another direction.

Additionally, the drawing shows that the sensor unit is detachably coupled to a carrier coupled with a needle and moves from a first position to a second position, but the sensor unit is detachably coupled to the plunger or a separate member provided on the plunger and can move together with the plunger.

In addition, the drawing shows that the moving member is elastically supported by the return member and is pushed and moved by the operating member, but the moving member may be configured to be moved by a separate driver that can be operated by the operating member.

In addition, the drawing shows that when the operating member is operated once, only the removing unit operates first to remove the protective sheet, and when the operating member is operated a second time, the insertion unit operates to insert the sensor into the skin of a user, but the applicator can be modified into a variety of different configurations. As another exemplary embodiment, the applicator may be configured so that both the insertion unit and the removing unit operate when the user operates the operating member once. In this case, after the separation operation of the protective sheet by the removing unit is completed, the sensor unit may move to the second position, or the separation operation of the protective sheet and the movement of the sensor unit may proceed together.

Additionally, the drawing shows that the operating member moves the moving member to operate the insertion portion and the removing unit, but the connection structure between the operating member and the insertion unit or the connection structure between the operating member and the removing unit is not limited to what is shown. That is, the insertion unit and the removing unit may be connected to the same operating member or different operating members in a variety of other ways other than linking to one moving member.

Additionally, as another exemplary embodiment, the base unit may simply take a configuration to support the sensor unit so that it is not separated from the skin of a user. In this case, a separate electronic unit that can process biometric information measured by the sensor unit and transmit it to an external terminal may be detachably coupled to the base unit. A separate electronic unit may be coupled to the base unit to be electrically connected to the sensor unit after the sensor unit is coupled to the base unit. A body attachable unit including such a base unit may be used by coupling a separate electronic unit.

Although the present disclosure has been shown and described in connection with preferred embodiments for illustrating the principles of the disclosure, the disclosure is not limited to the construction and operation as shown and described. Rather, those skilled in the art will understand that numerous changes and modifications can be made to the present disclosure without departing from the concept and scope of the attached registration claims.

The present invention may further include any one of the following items:
Item 1: An applicator for inserting a sensor for measuring biometric information into skin of a user, the applicator comprising: an applicator body having a bottom portion configured to be contactable with the skin of the user; an insertion unit installed to the applicator body to move the sensor unit, which comprises the sensor, a sensor unit housing to which the sensor is mounted, an adhesive layer comprised in the sensor unit housing, and a protective sheet covering the adhesive layer, from a first position where the sensor unit is spaced apart from the skin of the user to a second position where the sensor is inserted into the skin of the user; a carriage to which a part of the protective sheet is coupled and which is installed to the applicator body to move in a direction away from the sensor unit housing for releasing the protective sheet from the adhesive layer; and an operation member installed to the applicator body to operate the carriage by a manipulation of a user.
Item 2: The applicator according to item 1, wherein the carriage is installed to move in a direction away from the bottom portion.
Item 3: The applicator according to item 2, wherein the carriage is installed to move in a direction opposite to a movement direction of the sensor unit.
Item 4: The applicator according to item 1, comprising a carriage driver configured to move the carriage.
Item 5: The applicator according to item 1, comprising an elastic member configured to apply an elastic force to the carriage in a direction away from the bottom portion.
Item 6: The applicator according to item 5, further comprising a moving member installed to the applicator body to constrain movement of the carriage by engaging with the carriage such that the carriage maintains a third position in which the carriage is coupled to the protective sheet in a state in which the elastic member is elastically deformed, wherein the operating member is configured to operate to bias the moving member in a direction of being disengaged from the carriage.
Item 7: The applicator according to item 6, wherein the moving member is installed to move in a direction intersecting the moving direction of the carriage.
Item 8: The applicator according to item 6, further comprising a return member elastically deformably installed to the applicator body and configured to apply an elastic force in a direction of being retreated with respect to the moving member by being elastically deformed by the moving member advanced by the operating member, wherein the carriage is configured to, as the moving member advances by the operating member, be disengaged from a first detent portion provided at one side of the moving member to move to a fourth position engaged with a second detent portion provided at an other side of the moving member, and, as the moving member retracts by the return member, be disengaged from the second detent to move to a fifth position separating the protective sheet from the adhesive layer.
Item 9: The applicator according to item 8, wherein the insertion unit comprises a plunger installed movably from the first position to the second position together with the sensor unit, a needle separatably coupled to the sensor unit to be insertable into the skin of the user together with the sensor, a carrier installed to the plunger and coupled to the needle, and a plunger driver configured to provide a moving force in a direction of moving from the first position to the second position with respect to the plunger, and the plunger is configured to contact the moving member at the first position such that movement of the plunger is restricted.
Item 10: The applicator according to item 9, wherein a first moving distance by which the moving member advances to disengage the carriage from the first detent portion is shorter than a second moving distance by which the moving member advances to disengage the plunger from the moving member.
Item 11: The applicator according to item 10, wherein the carriage is configured to restrain a forward movement of the moving member to prevent the moving member from moving by the second moving distance in a state that the carriage is engaged with the second detent portion.
Item 12: The applicator according to item 9, wherein the plunger is configured to be released from the moving member after a separation operation of the protective film by the carrier is completed.
Item 13: The applicator according to item 9, wherein the applicator body includes a column portion accommodating the plunger, the column portion has a column rail configured to guide the carriage to move linearly, and the elastic member is installed to connect the column portion and the carriage.
Item 14: The applicator according to item 9, wherein the applicator body comprises a frame base portion having the bottom portion, a base frame protruding from the frame base portion and having a column portion accommodating the plunger, and a middle frame comprising a stage arranged at an upper side of the frame base and supporting the moving member, and a middle frame opening formed at a middle of the stage to allow the column portion to be inserted.
Item 15: The applicator according to item 9, further comprising a needle release driver configured to provide a moving force to the carrier such that the carrier moves the needle in a direction away from the skin of the user after the needle is inserted into the skin of the user with the sensor.
Item 16: The applicator according to item 1, wherein the protective sheet comprises a protection portion covering the adhesive layer, and a wing portion extending from an edge of the protection portion to be coupled to the carriage.
Item 17: The applicator according to item 1, wherein the protective sheet has a protective sheet groove, and the carriage comprises a grip portion inserted into the protective sheet groove.
Item 18: The applicator according to item 1, wherein the carriage is configured to operates to complete a separation operation of the protective sheet before the sensor unit moves from the first position.
Item 19: An applicator for inserting a sensor for measuring biometric information into skin of a user, the applicator comprising: an applicator body having a bottom portion configured to be contactable with the skin of the user; an insertion unit installed to the applicator body to move the sensor unit, which comprises the sensor, a sensor unit housing to which the sensor is mounted, an adhesive layer comprised in the sensor unit housing, and a protective sheet covering the adhesive layer, from a first position where the sensor unit is spaced apart from the skin of the user to a second position where the sensor is inserted into the skin of the user; and a grip portion installed to the applicator body such that a portion of the protective sheet is coupled, wherein the protective sheet is separated from the sensor unit housing by changing a distance between the sensor unit housing and the grip portion.
Item 20: The applicator according to item 19, wherein the grip portion is installed to the applicator body such that the grip portion is movable in a direction away from the sensor unit housing.
Item 21: The applicator according to item 19, wherein the grip portion is fixed at a position spaced apart from the bottom portion.
Item 22: An applicator for inserting a sensor into skin of a user, the applicator comprising: an applicator body to which a base unit comprising a base unit housing and an adhesive portion provided on the base unit housing to be attached to the skin of the user is separatably coupled, the applicator body having a bottom portion configured to be contactable with the skin of the user; an insertion unit installed to the applicator body to move the sensor unit, which comprises the sensor, a sensor unit housing to which the sensor is mounted, an adhesive layer comprised in the sensor unit housing to be adhered to the base unit housing, and a protective sheet covering the adhesive layer, from a first position where the sensor unit is spaced apart from the base unit to a second position where the sensor unit is coupled to the base unit such that the sensor is inserted into the skin of the user; a carriage to which a part of the protective sheet is coupled and which is installed to the applicator body to move in a direction away from the bottom portion for releasing the protective sheet from the adhesive layer; and an operation member installed to the applicator body to operate the carriage by a manipulation of a user.
Item 23: An applicator assembly comprising: a sensor unit comprising a sensor, a sensor unit housing to which the sensor is mounted, an adhesive layer comprised in the sensor unit housing, and a protective sheet covering the adhesive layer; an applicator body having a bottom portion configured to be contactable with skin of an user; an insertion unit installed to the applicator body to move the sensor unit from a first position where the sensor unit is spaced apart from the skin of the user to a second position where the sensor unit is inserted into the skin of the user; a carriage to which a part of the protective sheet is coupled and which is installed to the applicator body to move in a direction away from the bottom portion for releasing the protective sheet from the adhesive layer; and an operation member installed to the applicator body to operate the carriage by a manipulation of a user.
Item 24: The applicator assembly according to item 23, further comprising a base unit comprising a base unit housing and an adhesive portion provided on the base unit housing to be attached to the skin of the user, wherein the base unit separatably coupled to the applicator body, wherein the sensor unit housing is attached to the base unit housing by the adhesive layer at the second position.

## Claims

1. An applicator (30) for inserting a sensor (110) into skin of a user, the applicator (30) comprising:
an applicator body (300) having a bottom portion (312);
an insertion unit (400) installed to the applicator body (300) to move a sensor unit (100), which comprises the sensor (110), from a first position where the sensor unit (100) is spaced apart from the skin of the user to a second position where the sensor (110) is inserted into the skin of the user;
a carriage (610) installed to the applicator body (300);
an operating member (345) installed to the applicator body (300) to operate the carriage (610); and
a first elastic member (631) and a second elastic member (481) each exerting an elastic force in a compressed state .

2. The applicator (30) according to claim 1, wherein the first elastic member (631) exerts the elastic force in a direction away from the bottom portion (312) of the applicator body (300), and
wherein the second elastic member (481) has the elastic force in a direction of moving from the first position to the second position.

3. The applicator (30) according to claim 1 or 2, wherein the insertion unit (400) comprises:
a plunger (410) installed movably from the first position to the second position together with the sensor unit (100),
a needle (450) separatably coupled to the sensor unit (100) to be insertable into the skin of the user together with the sensor (110),
a carrier (422) installed to the plunger (410) and coupled to the needle (450), and
a plunger driver (480) configured to provide a moving force in a direction of moving from the first position to the second position with respect to the plunger (410).

4. The applicator (30) according to any one of the preceding claims, further comprising:
a third elastic member (486) having an elastic force such that the carrier (422) moves the needle (450) in a direction away from the skin of the user after the needle (405) is inserted into the skin of the user with the sensor (110).

5. The applicator (30) according to claim 4, wherein the third elastic member (486) is in the form of a tension spring.

6. The applicator (30) according to any one of the preceding claims, wherein the sensor unit (100) further comprises a sensor unit housing (120) to which the sensor (100) is mounted, an adhesive layer (155) provided on the sensor unit housing (120), and a protective sheet (160) covering the adhesive layer (155), and
wherein the applicator (30) further comprises a moving member (500) installed to the applicator body (300) to constrain movement of the carriage (610) by engaging with the carriage (610) such that the carriage (610) maintains a third position in which the carriage (610) is configured to be coupled to the protective sheet (160) in a state in which the first elastic member (631) is elastically deformed.

7. The applicator (30) according to claim 6, further comprising
a return member (350) elastically deformably installed to the applicator body (300) and configured to apply an elastic force in a direction of being retreated with respect to the moving member (500) by being elastically deformed by the moving member (500) advanced by the operating member (345).

8. The applicator (30) according to claim 6 or 7, wherein the protective sheet (160) comprises a protection portion (161) covering the adhesive layer (155), and a wing portion (164) extending from an edge of the protection portion (161) to be coupled to the carriage (610).

9. The applicator (30) according to any one of claims 6 to 8, wherein:
the protective sheet (160) has a protective sheet groove (167), and
the carriage (610) comprises a grip portion (616) inserted into the protective sheet groove (167).

10. The applicator (30) according to any one of claims 6 to 9, wherein the operating member (345) is configured to operate to bias the moving member (500) in a direction of being disengaged from the carriage (610).

11. The applicator (30) according to any one of claims 6 to 10, wherein the carriage (610) is configured to operate to complete a separation operation of the protective sheet (160) before the sensor unit (100) moves from the first position.

12. The applicator (30) according to any one of claims 3 to 11, insofar as being dependent upon claim 3, wherein the applicator body (300) includes a column portion (318) accommodating the plunger (410),
wherein the column portion (318) has a column rail configured to guide the carriage (610) to move linearly, and
wherein the first elastic member (631) is installed to connect the column portion (318) and the carriage (610).

13. The applicator (30) according to claim 12, wherein the applicator body (300) comprises:
a frame base portion (311) having the bottom portion (312),
a base frame protruding from the frame base portion (311) and having a column portion (318) accommodating the plunger (410), and
a middle frame (330) comprising a stage (333) arranged at an upper side of the frame base portion (311) and supporting the moving member (500), and a middle frame opening (334) formed at a middle of the stage (333) to allow the column portion (318) to be inserted.

14. The applicator (30) according to any one of claims 3 to 5, insofar as being dependent upon claim 3, wherein the applicator (30) further comprises a moving member (500) installed to the applicator body (300) to constrain movement of the carriage (610) by engaging with the carriage (610) such that the carriage (610) maintains a third position in which the carriage (610) is coupled to the protective sheet (160) in a state in which the elastic member (631) is elastically deformed, and
wherein the plunger (410) is configured to be released from the moving member (500) after a separation operation of a protective sheet (160) of the sensor unit (100) by the carrier (422) is completed.

15. The applicator (30) according to any one of the preceding claims, further comprising:
a carriage driver (630) configured to move the carriage (610).
